# EUROPÄISCHE PATENTSCHRIFT

(11) **EP 0 732 132 B1**
(45) Veröffentlichungstag und Bekanntmachung des Hinweises auf die Patenterteilung: **23.08.2000**
(21) Anmeldenummer: 96108321.9
(22) Anmeldetag: 26.10.1992
(51) Int. Cl.: A63J 23/02, A61L 9/12, G03B 31/00

(54) **Verfahren und Vorrichtung für die ereignisbezogene oder szenengenaue Duftdarbietung bei kinematografischen Aufführungen und dergleichen**
Process and device for diffusing perfumes that accurately correspond to events or scenes during cinematographic representation or the like
Procédé et dispositif de diffusion d'odeurs en rapport avec des événements ou des scènes déterminées pendant des représentations cinématographiques et similaires

(30) Priorität: 30.10.1991 DE 4135796
(43) Veröffentlichungstag der Anmeldung: 18.09.1996
(62) Teilanmeldung aus: 92922313.9
(73) Patentinhaber: Wittek, Götz-Ulrich, London W1R 5RD (GB)
(72) Erfinder: Wittek, Götz-Ulrich, London W1R 5RD (GB)
(74) Vertreter: DIEHL GLAESER HILTL & PARTNER

(56) Entgegenhaltungen:
- DE-A- 4 033 076
- US-A- 3 795 438
- US-A- 4 603 030
- US-A- 4 629 604

## Beschreibung

Die Erfindung betrifft ein Verfahren zur Erhöhung der sinnlichen Wahrnehmung von visuellen und/oder akustischen Darbietungen, insbesondere bei Diavorträgen, Videos, Fernsehsendungen, Hörspielen und dergleichen, sowie eine Vorrichtung zur Durchführung eines derartigen Verfahrens.

Bezweckt wird mit der Erfindung, den Zuschauer und/oder Zuhörer wesentlich stärker in das Geschehen miteinzubeziehen und dadurch das Unterhaltungserlebnis in seiner Faszination außerordentlich zu steigern, wobei vorrichtungsseitig, insbesondere im öffentlichen Bereich, möglichst geringe Kosten zur Realisierung des Systems erforderlich sein sollen.

Die Erfindung geht von der Erkenntnis aus, daß visuelle und/oder akustische Darbietungen dadurch in ihrer Wirkung auf Zuschauer bzw. Zuhörer gesteigert werden können, wenn auch der Geruchssinn in den Bereich der sinnlichen Wahrnehmung involviert wird.

Generell ist es schon bekannt, große Räume, Säle und dergleichen allmählich mit Duft zu versehen, wie dies seit Jahrhunderten beispielsweise von den christlichen, hinduistischen und buddhistischen Kirchen praktiziert wird. Hierbei handelt es sich um eine Maßnahme, die dazu beitragen soll, die innere Beteiligung der anwesenden Gläubigen zu steigern. Das Zuführen der Düfte erfolgt dabei in der Regel durch eine oder zwei zentrale Räucherlampen oder dergleichen, wobei sich der Duft von Sandelholz, Weihrauch oder dergleichen allmählich über den Raum ausbreitet und in diesem ständig verbleibt, bis schließlich nach Beendigung der Zeremonie ein Lüften des Raumes erfolgt.

Bekannt geworden ist weiterhin, daß im Zuge der sogenannten "Aroma-Therapie" versucht wird, Büros oder Krankenhäuser über die vorhandene Klimaanlage allmählich mit Duft zu versehen, wie das beispielsweise die "Kajima AG" in Tokyo oder das "Memorial-Sloan-Kettering-Hospital", New York, praktizieren. Dabei geht es darum, gewisse Stimulations- bzw. Motivationseffekte zu erzielen, beispielsweise Limonenduft am Morgen zur Vermittlung von Frische und Aktivität. Es handelt sich um therapieähnliche Maßnahmen, die nicht dazu dienen und auch nicht dazu vorgesehen sind, die Wirkung sinnlicher Wahrnehmung von visuellen und/oder akustischen Darbietungen gezielt zu erhöhen.

Bekannt ist ferner ein im wesentlichen manuelles und allmähliches Verteilen von einigen wenigen Duftnoten bei Theater- und Musicalaufführungen, beispielsweise bei den Aufführungen des Musicals "HAIR" im Jahre 1969. Hierbei ging es darum, die Wirkung von visuellen und/oder akustischen Darbietungen zu unterstützen, indem, ähnlich wie bei dem zuvor beschriebenen kirchlichen Bereich, ein bestimmter Duft von einer zentralen Stelle aus freigegeben wurde und sich allmählich unkontrolliert über den Zuschauerraum verbreitete. Ausgelöst wurde dieser Geruchsbeitrag zwar als Begleitung von jeweils mehreren Szenen, erreichte jedoch die Zuschauer häufig insgesamt erst zu einem späteren Zeitraum und verblieb dabei im Zuschauerraum, ohne daß irgendein erkennbarer Bezug zur weiteren Szenenfolge existierte.

Bekannt geworden ist ferner ein 1981 durchgeführter Versuch, bei den Vorführungen des Films "Polyester" (Regisseur: John Waters) zusammen mit der Eintrittskare eine "Rubbelkarte" mit vier durchnummerierten und erst durch Reiben freisetzbaren Gerüchen auszugeben. Auf der Leinwand erschien dann während der Vorführung an bestimmten Stellen eine Zahl, worauf das Rubbelkärtchen dann vom Zuschauer an der mit derselben Zahl gekennzeichneten Stelle gerieben werden mußte. Durch dieses manuelle Reiben wurde schließlich ein bestimmter (hier: unangenehmer) Geruch freigesetzt, der gemäß dem "Lexikon des internationalen Films", Bd. 6, S. 2973, Reinbeck bei Hamburg, 1987, "... ein die Nase beleidigender Gag" war. Nachteilig ist bei diesem Verfahren, daß jeder Zuschauer bestimmte Gerüche jeweils selbst manuell freisetzen mußte und dadurch in seiner Konzentration von dem visuellen und akustischen Geschehen deutlich abgelenkt war, zumal die Geruchskarte vor die Nase gehalten werden mußte. Hinzu kamen Störungen der Darbietungen durch verständliche Unruhen im Zuschauerraum, die eher einer Erhöhung der gesamten sinnlichen Wahrnehmung abträglich waren.

Aus der US-A-4,629,604 ist eine Dufterzeugungsvorrichtung bekannt, welche mit einer Patrone, die mehrere Düfte trägt, beschickt wird. Die Patrone ist aus einer ebenen Anordnung gleichartiger Rahmenelemente, welche in einem Rahmenabschnitt eingepaßt sind, aufgebaut. Jedes Rahmenelement weist ein Kissen aus absorbierendem Material auf, welches zwischen den Rändern des Rahmenelements eingespannt ist, so daß ein zentraler Bereich des Kissens freigelassen ist. Das Kissen jedes Rahmenelements ist mit einem Duft imprägniert, der sich von den anderen Düften unterscheidet. Wenn die Patrone in einem Schlitz der Dufterzeugungsvorrichtung eingeschoben wird, liegt diese Patrone über einer komplementären Bienenwabenstruktur, wobei jede Bienenwabenzelle paßgenau unter einem Rahmenelement zu liegen kommt. Jede Zelle enthält eine individuelle, elektrische Heizeinrichtung, so daß beim Einschalten einer bestimmten Heizeinrichtung die Luft in der entsprechenden Zelle erwärmt wird, wodurch sich ein Überdruck ausbildet, welcher bewirkt, dass die erwärmte Luft durch den zentralen Bereich des Kissens gedrückt wird unter Verflüchtigung des flüssigen Duftstoffs. Der erzeugte aromatisierte Dampf wird dann durch in der Vorrichtung angeordnete Gebläse an die Umgebungsluft abgegeben. Die abzugebenden Düfte können entweder manuell eingestellt oder mit den Szenen eines Videos oder einer Filmdarbietung synchronisiert werden.

Der Erfindung liegt die Aufgabe zugrunde, ein Verfahren und eine Vorrichtung der eingangs genannten Art zu schaffen, mit welchen verschiedenartige visuelle und/oder akustische Ereignisse in ihrer unterschiedlichen individuellen Wirkung ohne Beeinträchtigung der Konzentration des Zuschauers bzw. Zuhörers durch eigene Mitwirkung verstärkt werden.

Erfindungsgemäß wird diese Aufgabe verfahrensseitig durch die im Patentanspruch 1 gekennzeichneten Merkmale und vorrichtungsseitig durch die im Patentanspruch 13 gekennzeichneten Merkmale gelöst. Bevorzugte weitere Ausgestaltungen der Erfindung, die das Verfahren bzw. die Vorrichtung vorteilhaft weiterbilden, sind den jeweils nachgeordneten Patentansprüchen zu entnehmen.

Aufgrund des erfindungsgemäßen Verfahrens bzw. der erfindungsgemäßen Vorrichtung wird in vorteilhafter Weise erstmals erreicht, verschiedene Düfte sowohl zeitlich als auch bezüglich der Zuschauer bzw. Zuhörer örtlich sehr genau zu einer zugehörigen Filmszene, Musiksequenz etc. passend wahrnehmbar zu machen, ohne daß hierzu eine Mitwirkung des Zuschauers bzw. Zuhörers erforderlich ist. Dabei werden in günstiger Weise unterschiedliche Düfte ereignisentsprechend und synchron derart vermittelt, daß die suggestive und emotionale Wirkung von derartigen Ereignissen einen außerordentlich großen Realitätsbezug erhält und den Zuschauern bzw. Zuhörern ein Miterleben im ursprünglichen Sinne ermöglicht.
Hierzu trägt in günstiger Weise eine Vielfalt von unterschiedlichen Düften aus einem Duftvorrat bei, wobei die zugeführten Düfte in ihrer Intensität vorzugsweise an die einzelnen Ereignisse graduell angepaßt werden. Ermöglicht wird weiterhin vorteilhaft ein realitätsnaher Wechsel von zugeführten Düften mit jeweils zeitlich abnehmender bzw. zunehmender Intensität im Sinne einer Überblendung sowie auch in bestimmten Fällen eine Mischung oder Überlagerung verschiedenartiger Düfte. Wenn beispielsweise auf der Leinwand Jean Gabin in einem Auto zuerst über eine Wiese und anschließend zum Meer fährt, wird zunächst Wiesen- und Grasgeruch spürbar und anschließend der bekannte Meerwassergeruch eingespielt, oder wenn etwa in dem Film "The Sheltering Sky" John Malkovich die Schauspielerin Debra Winger in einem verfallenen Hotel in Tanger auf den Hals küßt, wird eine verwirrende Mischung aus orientalischem Parfüm, süßlichem Hautduft und dem modrigen Grundton des Hotelzimmers wahrnehmbar.

Die passenden Düfte werden jedem Zuschauer bzw. Zuhörer entweder gruppenweise oder separat zugeführt, wobei vorzugsweise nach einer weiteren Ausgestaltung der Erfindung zugeführte Düfte während und/oder nach den zugeordneten Darbietungsereignissen gruppenweise oder separat abgeführt werden.

Die erfindungsgemäße Vorrichtung weist vorteilhaft eine Einrichtung zur individuellen Aufnahme und Abgabe verschiedener Düfte und eine zugeordnete Einrichtung zur Ansteuerung individueller Duftabgaben und -zuführungen abhängig von individuellen visuellen und/oder akustischen Ereignissen auf, wobei bevorzugt die Duftaufnahme- und -abgabeeinrichtung mehrere unterschiedliche Duftvorräte aufweist, die mittels einer Auswahleinrichtung selektiv für die Duftzuführung freigebbar sind. Hierzu liegen die Duftvorräte bevorzugt in freigebbarer fester oder flüssiger Form vor und sind durch Kontaktierung an einen passierenden Luftstrom abgebbar. Alternativ können die Duftvorräte gasförmig oder in Fluid- bzw. Aerosolform vorliegen und unter Druck freigebbar sein. In beiden Fällen ist gewährleistet, daß Düfte nur gezielt und dann schnell und wirkungsvoll zur Verfügung stehen.
Zur Aufnahme der Duftvorräte ist nach einer bevorzugten Ausgestaltung der Erfindung wenigstens eine Duftschaltbox oder eine Duftdiskette oder eine Duftrollenanordnung vorgesehen, wobei die Duftschaltbox entweder im Bereich der Ansteuerungseinrichtung oder im Zuschauerbzw. Zuhörerbereich angeordnet ist. Vorzugsweise weist die Duftaufnahme- und -abgabeeinrichtung weiterhin eine Duftintensitätsregelung auf, um für Zuschauer bzw. Zuhörer eine Abstimmung auf die indivuelle Geruchsempfindlichkeit vornehmen zu können.
Nach einer bevorzugten Ausgestaltung der Erfindung weist die Duftaufnahme- und -abgabeeinrichtung für das Einbringen des jeweiligen Duftes in einen Luftstrom ein spiralförmiges, sich unter Berücksichtigung von Druckverhältnissen leicht konisch verjüngendes Leitungssystem mit Ein- und Auslaßsteuerelementen und zugeordneter Bypassleitung auf. Alternativ kann die Duftaufnahme- und -abgabeeinrichtung für das Einbringen des jeweiligen Duftes in einem Duftstrom wenigstens zwei Kontaktierflächen für zugeordnete Teile von Luftströmen aufweisen. Nach einer weiteren Alternative kann die Duftaufnahme- und -abgabeeinrichtung des Duftmischers für mehrere Duftgrundkomponenten ausgebildet sein, die in durch Einzelluftströme ansteuerbaren Duftmischrollen vorgesehen sind.

Die vorgenannten Alternativen der Duftaufnahme- und -abgabeeinrichtungen sind insbesondere im Zusammenhang mit Duftdisketten oder einem Duftmischer als mobile Einheit vorgesehen.

Bei der Ausbildung der Duftaufnahme- und -abgabeeinrichtung als Duftmischer ist nach einer weiteren Ausgestaltung der Erfindung die Reihenfolge der Kontaktierung der Duftmischrollen abhängig von deren Duftstärke derart festgelegt, daß die stärkste Duftkomponente zuletzt kontaktiert wird, wobei der Duftmischer ein Gebläse zur Luftstromerzeugung aufweist. Geschützt sind die Duftvorräte gegen Duftverlust durch zu öffnende Abschlußeinrichtungen und/oder temperaturabhängige Duftfreisetzungen, wobei im letzteren Fall nur in bestimmten Temperaturbereichen eine Duftabgabe erfolgt.

Die für die Erfindung wesentliche Einrichtung zur Ansteuerung individueller Duftabgaben und -zuführungen weist eine Erfassungseinrichtung für individuelle ereignissynchrone Duftsteuersignale auf, welche vorzugsweise zur Aufname von optischen und/oder elektrischen Duftsteuersignalen ausgebildet ist, welche kodierte Informationen über Duftart und/oder Duftzusammensetzungen und/oder Duftvorratschalter und/oder Häufigkeit der Verwendung des Duftvorrates sowie Duftzuführdauer enthalten. Des weiteren können die Duftsteuersignale bevorzugt Informationen zur Duftintensität, zum Duftzuführbeginn und -zuführende sowie zu einem Duftwechsel enthalten. Als Duftsteuersignale werden kodierte Filmspuren oder ereignisgekoppelte Signalgeber bevorzugt.

Die Einrichtung zur Ansteuerung indivudeller Duftabgaben und -zuführungen weist eine Druckluftquelle auf, die mit der Einrichtung zur individuellen Aufnahme und Abgabe verschiedener Düfte und einem diesem nachgeordneten Duftverteilsystem verbunden ist. Die Druckluftquelle besteht dabei in günstiger Weise entweder aus einem Drucklufterzeuger bzw. Kompressor oder einem Druckluftspeicher. Das Duftverteilsystem weist vorzugsweise steuerbare Duftauslässe für Sitzgruppen und/oder Einzelsitze auf und ist bevorzugt mit Haupt- und Zwischenverteilern versehen, um eine synchrone Duftzuführung zu gewährleisten.

Nach einer bevorzugten weiteren Ausgestaltung der Erfindung ist dem Duftverteilsystem ein Duftabzugssystem für Einzelsitze und/oder Sitzgruppen zugeordnet, um die Einzelwirkung unterschiedlicher Düfte in ihrer zeitlichen Reihenfolge zu erhöhen.

Das Duftverteilsystem ist vorzugsweise mit dünnen Rohrleitungen ausgebildet, deren Länge und Durchmesser auf die Verteilsituation im Zuschauer- bzw. Zuhörerraum abgestimmt ist, wobei bevorzugt die Rohrleitungen als Doppelleitungen mit Zu- und Rückführung für eine sitzindividuelle Duftintensitätsregelung ausgebildet sind.

Gemäß weiterer Ausbildung werden die Düfte in Form eines mit Duftstoffen angereicherten Duft-Luft-Gemischstromes zugeführt, der mit Bestandteilen sehr niedriger spezifischer Dichte angereichert wird, derart, daß das austretende Duft-Luft-Gemisch leichter als die Umgebungsluft ist.

In besonders vorteilhafter Weise wird der austretende Duft-Luft-Gemischstrom in eine in Strömungsrichtung fortschreitende rotierende Wirbelbewegung versetzt, um so über eine vorgegebene Länge nach dem Austritt einen gerichteten Strömungsschlauch zu bilden.

Hierzu werden in besonders vorteilhaftr Weise im Bereich der Austrittsöffnung wendelförmig angeordnete Elemente, insbesondere wendelförmig angeordnete Röhrchen, vorgesehen. Hierdurch wird noch im Bereich der Austrittsöffnung innerhalb des Austrittsrohres durch die wendelförmig angeordneten Röhrchen ein Duft-Luft-Gemisch-Wirbelstrom erzeugt, der gleichsam spiralartig nach außen tritt.

In vorteilhafter Weise sind an jedem Platz jeweils zwei Austrittsöffnungen so angeordnet, daß die beiden rotierenden Duft-Luft-Gemischströme eine gegenläufige Drehbewegung ausführen und sich in ihrer Drehbewegung unterstützen, um so zusätzlich einen resultierenden, tangentialen, gerichteten Duft-Luft-Gemischstrom zu erzeugen.

Gemäß einer besonders vorteilhaften Ausgestaltung sind die Duftauslässe der Sitzgruppen und/oder Einzelsitze zur Einstellung der Austrittsöffnung des Duft-Luft-Stromes schwenkbar gelagert. Vorzugsweise werden Kugelgelenklager eingesetzt.

Weitere Einzelheiten, Vorteile und Merkmale der Erfindung sind dem anschließenden Beschreibungsteil zu entnehmen, in dem die Erfindung unter Bezugnahme auf die beigefügten Zeichnungen näher erläutert wird.

Im folgenden werden anhand der beiliegenden Zeichnungen Ausführungsbeispiele der Erfindung näher beschrieben.

Es zeigen:
- Fig. 1: einen schematischen Überblick über ein nicht erfindungsgemäßes Ausführungsbeispiel eines Duftkinos,

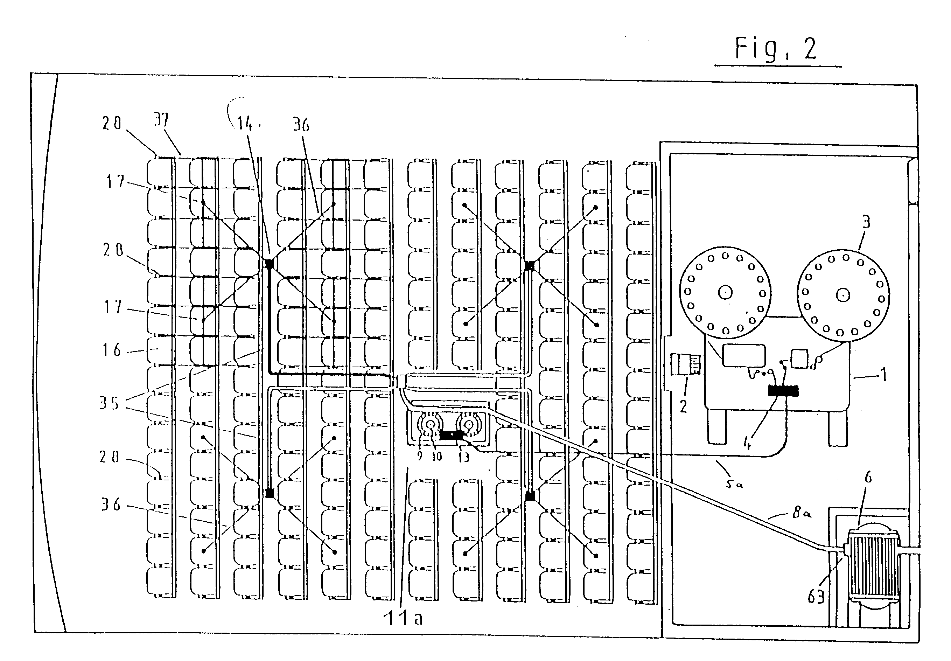

- Fig. 2: einen schematischen Überblick über ein weiteres nicht erfindungsgemäßes Ausführungsbeispiel eines Duftkinos,
- Fig. 3: eine schematische Darstellung von Filmmaterial (12) des Ausführungsbeispieles in Fig. 1 mit Duftimpulsen,
- Fig. 4: eine Draufsicht mit Teilschnitt einer Duftschaltbox (7) des Ausführungsbeispiels in Fig. 1,
- Fig. 5: eine seitliche Draufsicht mit Teilschnitt der Duft-schaltbox (7) aus Fig. 4 mit Duftüberblend-Einrichtung,
- Fig. 6: einen Längsschnitt durch einen Duftanschluß (25) und durch einen Duftträger (10) des Ausführungsbeispiels in Fig. 4, 5 und 1,
- Fig. 7: eine Draufsicht auf eine getrennte Duftüberblend-Einrichtung eines weiteren Ausführungsbeispiels,
- Fig. 8: eine Ansicht einer zu allen Kinositzen verlegten Doppel leitung (45) des Ausführungsbeispiels in Fig. 1 und 11 - in Originalgröße -,
- Fig. 9: einen Längsschnitt eines Sitzplatz-Duftreglers (72) des Ausführungsbeispiels in Fig. 1, 8 und 11,
- Fig. 10: einen Längsschnitt des Sitzplatz-Duftreglers (72) aus Fig. 9 in Schließstellung,
- Fig. 11: eine schematische Darstellung eines Kinositzes (16) eines Duftkinos,
- Fig. 12: eine Draufsicht auf zwei Duftträger (10) mit schematischer Darstellung eines Unterdruck-Duftanschlusses (25a) eines weiteren Ausführungsbeispiels,
- Fig. 13: eine Draufsicht auf eine Duftresorberleitung (58) eines weiteren Ausführungsbeispiels,
- Fig. 14: eine Draufsicht auf eine Duftresorberleitung (58a) eines weiteren Ausführungsbeispiels mit integrierter Einzelleitung 37,
- Fig. 15: eine schematische Darstellung eines Duftcomposers (50) eines weiteren Ausführungsbeispiels,
- Fig. 16: eine Draufsicht eines Duftdisketten-Laufwerks (55) eines weiteren Ausführungsbeispiels der Erfindung,
- Fig. 17: eine Draufsicht auf eine Film-Duftdiskette (57) des Ausführungsbeispiels aus Fig. 16.

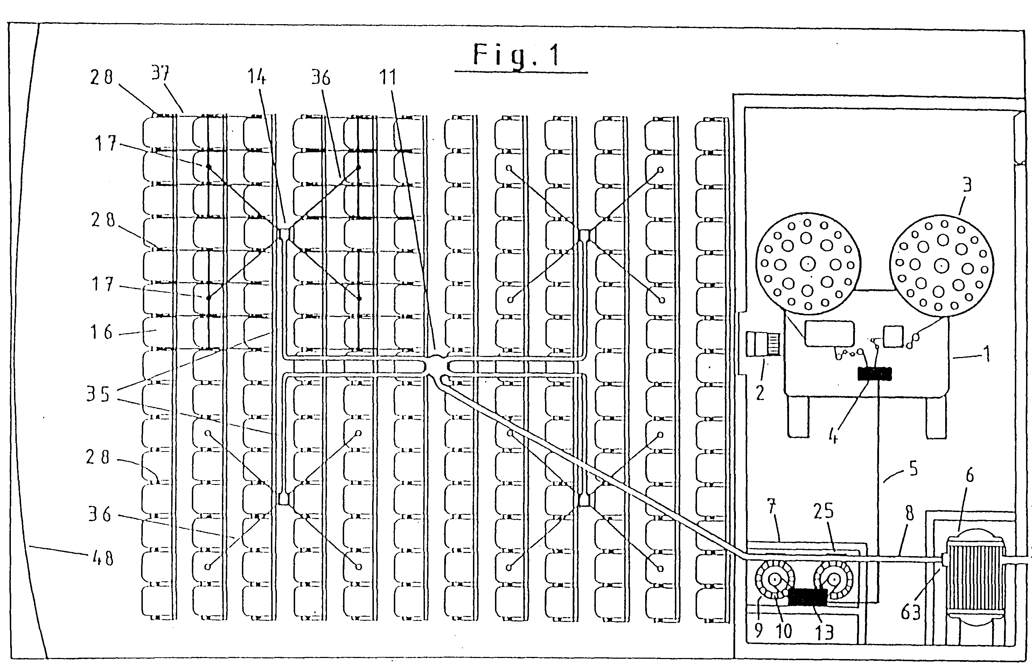

Das in den Figuren 1, 3, 4, 5, 6, 8, 9, 10 und 11 dargestellte Ausführungsbeispiel besteht aus einem in Fig. 1 dargestellten Filmprojektor 1, welcher neben den üblichen Ausstattungsmerkmalen, wie Projetionsobjektiv 2, Filmrollen 3 etc., zusätzlich mit einem Impulsabtaster 4 sowie einer speziellen Signalleitung 5 versehen ist.

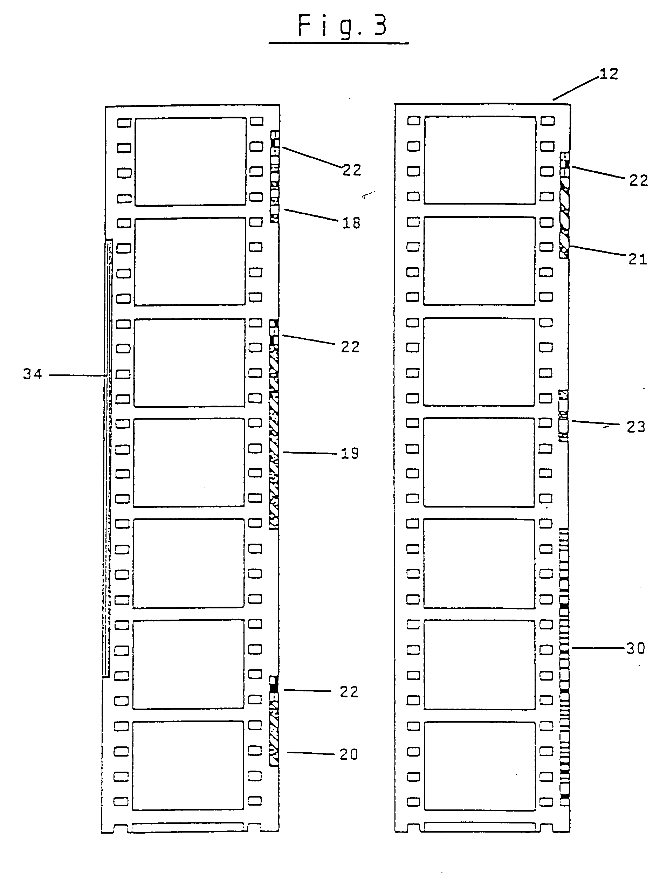

Der Impulsabtaster 4 ist dabei in der Lage, spezielle Steuerimpulse 18-22, die auf den Film 12 (Fig. 3) aufgebracht werden, zu lesen und in z.B. elektronische Signale umzuwandeln.

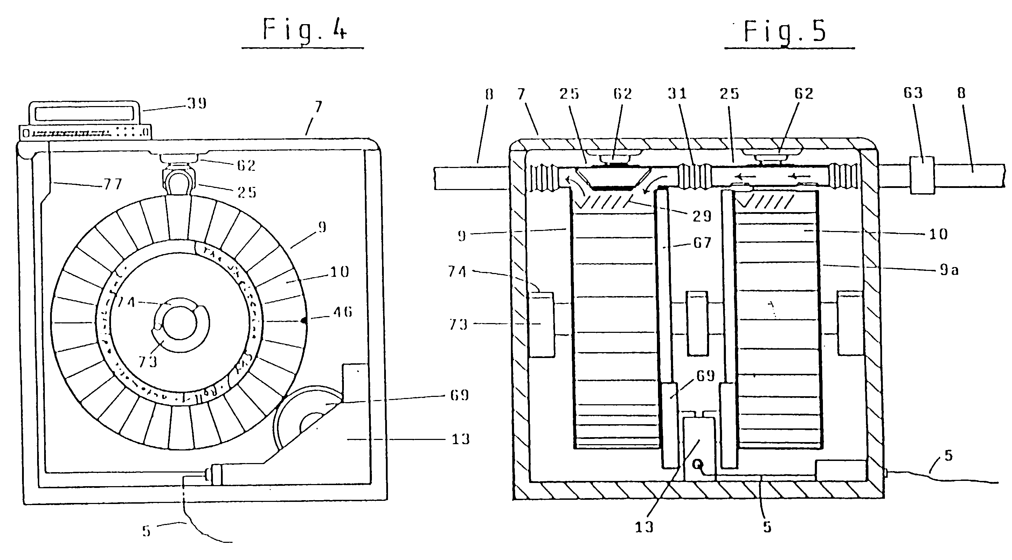

Die Signalleitung 5 verbindet nun den Impulsabtaster 4 mit einer Duft-Schaltbox 7, worin sich mehrere Duftrollen 9 und 9a mit einer Vielzahl von Duftträgern 10 sowie eine Duftrollensteuerung 13 befinden (Fig. 1, 4 u. 5).

Die Duft-Schaltbox 7 ist ihrerseits an eine Druckluftleitung 8 angeschlossen, die von einer Druckluftquelle, vorzugsweise von einem sehr leisen Elektrokompressor 6 o.ä.m., mit Druckluft versorgt wird. Die Druckluftleitung 8 führt nun von der Duft-Schaltbox 7 zu einem Hauptverteiler-Kasten 11, wo sie in mehrere Hauptleitungen 35 geteilt wird, die ihrerseits zu Zwischenverteilern 14 führen.

In den Zwischenverteilern 14 werden die Hauptleitungen 35 in kleinere Verteiler-Leitungen 36 unterteilt, die zu den Endverteilern 17 führen, wo sich die Verteiler-Leitungen 36 ein weiteres mal in noch kleinere Einzelleitungen 37 aufteilen.

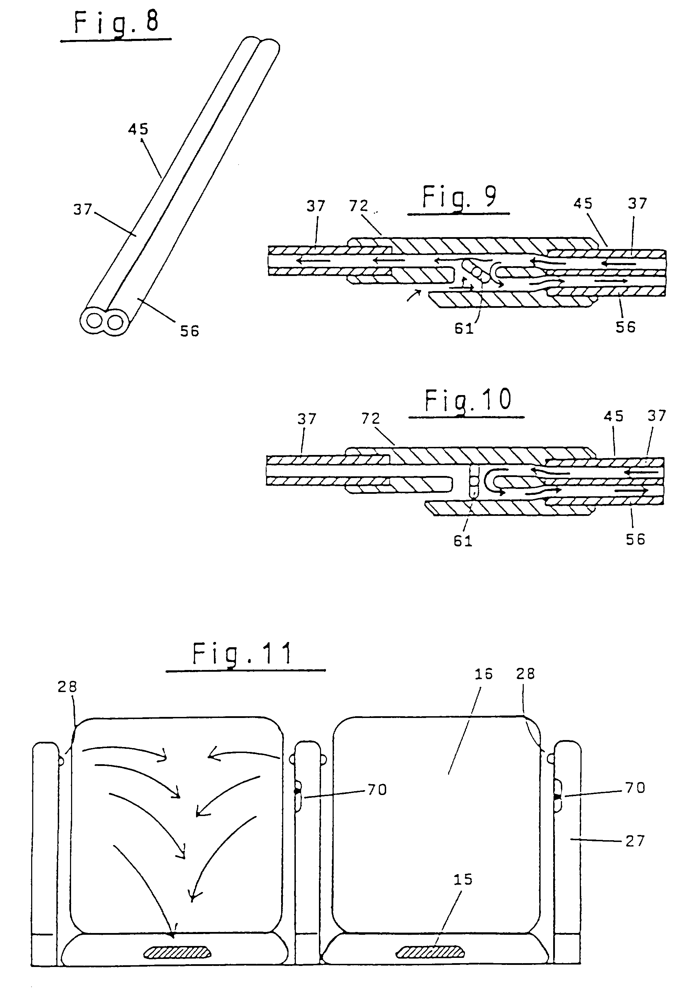

Die Einzelleitungen 37 führen schließlich zu den Duftarmlehnen 27 jedes einzelnen Kinositzes 16 und münden dort in einen Duftgeber 28 (Fig. 11).

Der rein schematische Betriebsablauf des Duftkinos vollzieht sich zunächst auf folgende Weise:

Während bei der Vorführung eines Kinofilms die üblichen optischen und akustischen Informationen über das Filmmaterial 12 an das Projektionsobjektiv 2 und die Tonköpfe des Filmprojektors 1 geleitet und auf die Leinwand 48 projiziert bzw. an die Lautsprecher weitergegeben werden, läuft der gezeigte Film 12 dabei vorzugsweise innerhalb des Projektors 1 gleichzeitig durch einen Impulsabtaster 4, welcher spezielle Informationen bezüglich einer möglichen Einleitung von Duftnoten abliest (Fig.1).

Die abgelesenen Duft-Impulse 18-22 (Fig.3) können dabei je nach Filmtyp z.B. in elektronischer, optischer etc. Form z.B. auf der Tonspur, einer Ausgleichsspur oder speziellen geeigneten Sonderspuren oder Film-Bestandteilen gespeichert werden.

Statt die Informationen über Duft-Einleitungen und u.U. die Steuerung weiterer Ergänzungen des Filmgeschehens über Impulse 18-22 zu steuern, ist es auch möglich, die entsprechenden Informationen über besondere, elektronisch lesbare Film-Barcodes 30 (Fig. 3) zu speichern, die von einem entsprechenden, nicht dargestellten Elektronik-Barcode-Filmscanner während der Filmvorführung vom laufenden Filmmaterial 12 abgelesen werden können.

Diese Film-Barcodes 30 sind somit nicht, wie sonstige Barcodes, optisch gespeichert, sondern vorzugsweise durch magnetische 3arcodes in nicht wahrnehmbaren Frequenzbereichen.
Auf diese Weise können sie u.U. als Meta-Information auf eine bereits bespielte Tonspur zusätzlich draufgelegt werden, ohne daß das Code-Signal hörbar wird oder der Ton der Tonspur irgendwie beeinträchtigt wird.

Auch die oben beschriebenen Duft-Impulse (18-22) können auf verschiedene Arten als Meta-Information auf bereits bestehendes Filmmaterial aufgebracht werden.

Hierdurch kann u.U. altes Filmmaterial ohne größere Veränderungen nachcodiert werden, so daß in alten Projektoren lediglich eine entsprechende Code- oder Impulslese-Einrichtung nachträglich installiert wird, ohne daß weitere technische Anpassungen größerer Art notwendig sind.

Eine weitere Alternative zu den Impuls-Befehlen 18-22 ist ein Steuer-Pilotsignal 34 (Fig. 3), welches einen Steuerbefehl nicht in der Form "ein" oder "aus" gibt, sondern deren Steuerbefehle nur wirksam sind, solange das Signal gegeben wird. Sobald das Signal abbricht, wird zugleich auch die gesteuerte Funktion beendet, was für einige der im folgenden beschriebenen Funktionsabläufe u.U von Vorteil sein kann.

Der gespeicherte Impuls 18-22 (Fig.3) wird nun über den Impulsabtaster 4 (Fig.1) abgelesen und dabei in ein z.B. elektronisches Signal umgesetzt und über die Signalleitung 5 an die Duftrollensteuerung 13 einer Duft-Schaltbox 7 weitergeleitet (Fig. 1, 4 und 5).
Die Duft-Schaltbox 7 ist ihrerseits an eine Druckluftleitung 8 angeschlossen, welche durch eine leise Druckluftquelle, z.B. durch einen sehr leisen Elektrokompressor 6, der sich innerhalb der Filmvorführerkabine befindet, gespeist wird (Fig. 1).

Das ankommende Steuersignal führt nun dazu, daß die DuftrollenSteuerung 13 den zu dem Signal passenden Duft auf der Duftrolle 9 ansteuert.

Hierdurch dreht sich die Duftrolle 9 ein kleines Stück, und zwar so, daß der entsprechende Duftträger 10 sich genau vor dem Duftanschluß 25 plaziert (Fig. 1, 4, 5 und 6).

Der Duftträger 10 wird hierbei speziell durch das Signal "Pre-Code" 18 angesteuert, während die Signale 19-21 andere Funktionen steuern, wie im folgenden näher beschrieben wird (Fig. 3).

Bei dem vorliegenden Ausführungsbeispiel der Druckluftleitung 8 (Fig.5) mit ihren Verzweigungen ist es hierbei vorteilhaft, wenn der Duftanschluß 25 nach Einschleusen des Duftträgers 10 mit einer Vorrichtung, der Andrück-Mechanik 62, leicht gegen die Duftrolle 9 bzw. den Duftträger 10 gedrückt wird.

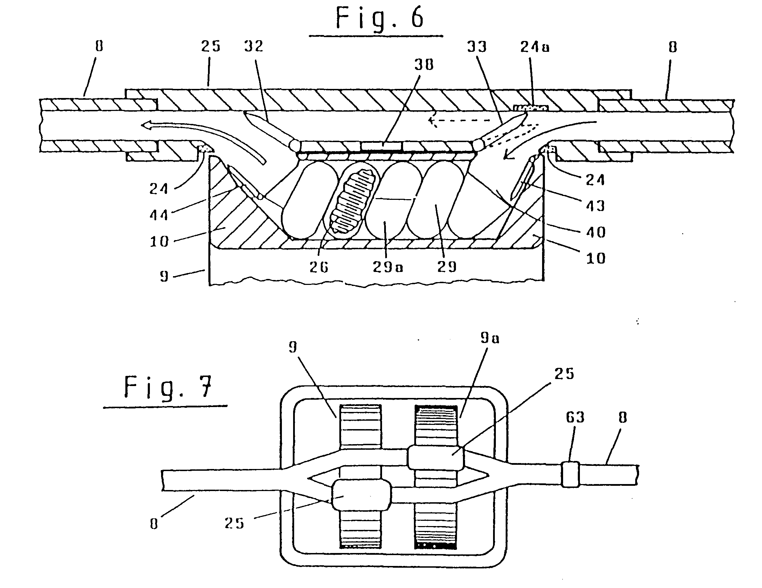

Hierbei sind die Berührungskanten des Duftanschlusses 25 dann vorzugsweise mit dichtenden Materialien 24 (Fig. 6) versehen, so daß sich ein geschlossenes, abgedichtetes System ergibt.

Während des Drückvorganges der Andruck-Mechanik 62 wird dabei der für die Drehbeweglichkeit der Duftrolle 9 notwendige, minimale Zwischenraum zwischen der Duftrolle 9 und dem Duftanschluß 25 überwunden (Fig. 4 und 5).

Der Zwischenraum zwischen Duftrolle 9 und Duftanschluß 25 bewegt sich dabei vorzugsweise bei 1 Millimeter.

Eine entsprechende Flexibilität der Druckluftleitung 8 wird in den meisten Fällen gegeben sein.

Falls die Druckluftleitung 8 aber aus sehr unflexiblem Material besteht oder sehr dick ist, wird die für den Drückvorgang notwendige Beweglichkeit der Druckluftleitung 8 in diesem Ausführungsbeispiel dann durch Einfügen von flexiblen Rohrmanschetten 31 vor und nach dem Duftanschluß gewährleistet (Fig. 5).

Nachdem der Duftträger 10 nun im Duftanschluß 25 verankert wurde, öffnet sich der Duftstärkeregler 33 ganz (oder teilweise, siehe den gestrichelten Duftstärkeregler), wobei sich gleichzeitig der Luftstromregler 32 gleich weit öffnet (Fig. 6).

Der Luftstrom der Druckluftleitung 8 wird nun zur Einlaßöffnung 40 des Duftträgers 10 hin geleitet (Fig. 6).

(Bei nur teilweiser Öffnung, siehe gestrichelter Duftstromregler 33 entsprechend 32, wird nach dem Öffnen des Duftstärkereglers 33, 32 weiterhin ein Teil der Luft am Duftträger 10 vorbeigeleitet, wie weiter unten noch näher beschrieben wird).

Der Duftträger 10 kann hierbei noch zusätzlich mit Dichtklappen 43 und 44 oder entsprechenden Ventilarten versehen sein (Fig. 6), die z.B. mit einer einfachen Metallfeder o.ä.m. verschließend vorgespannt sind und nun einfach dem andrückenden Luftstrom nachgeben, oder welche elektronisch geöffnet werden.

Eine weitere Möglichkeit besteht darin, daß die Dichtklappen 43, 44 beim Andrücken des Duftträgers 10 an den Duftanschluß 25 (Fig. 6) mechanisch durch einen nicht dargestellten Vorsprung o.ä.m. geöffnet werden.

Diese Dichtklappen 43, 44 können auch verhindern, daß die in den Duftträgern 10 gespeicherten Duftstoffe sich schon vor ihrer eigentlichen Benutzung verflüchtigen.

Außerdem kann damit verhindert werden, daß sich in der Duft-Schaltbox 7 ein unangenehm riechendes Sammelsurium von allen im Duftrad 9 gespeicherten Duftnoten bildet, welches dann bei der Einleitung von Duftnoten u.U. mit in den Luftstrom gelangen könnte.

Ob der Duftträger 10 mit zusätzlichen Dichtklappen 43, 44 versehen wird, hängt im wesentlichen von der Speicherart und Flüchtigkeit der Duftstoffe ab, so daß bei trägen Speichersystemen hierauf u.U. verzichtet werden kann.

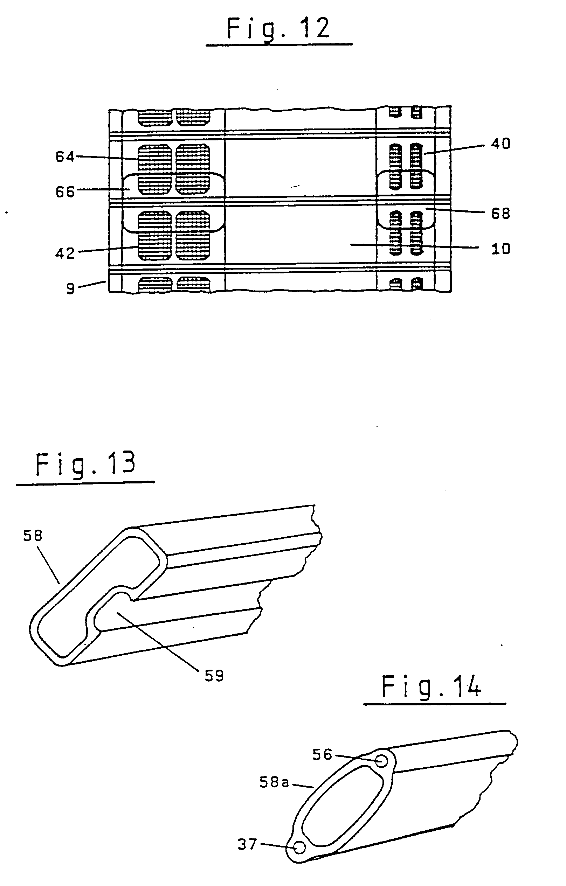

Bei derartigen, bei unbewegter Luft trägen Systemen eines weiteren Ausführungsbeispiels in Fig. 12, wo auf Dichtklappen 43, 44, verzichtet wird, würde der aus der Druckluftleitung 8 kommende Luftstrom nur sehr wenig in den Duftträger 10 geleitet werden. Vielmehr würde der Duftanschluß 25 hierbei so konstruiert werden, daß sich im Leitungsbereich über der Auslaßöffnung 42 ein gewisser Unterdruck bildet. Hierdurch würde dann die Luft aus der Druckluftleitung 8 gesaugt statt in sie hineingepreßt zu werden.

Der Vorteil wäre bei diesem System, daß u.U. einerseits auf die Dichtklappen 43, 44 verzichtet werden kann und nur ein sehr engmaschiges, sogenanntes Duftgitter 64 die Öffnungen bedeckt (Fig. 12).
Des weiteren könnte bei dem Ausführungsbeispiel in Fig. 12 auch auf die Andrück-Mechanik 62 und das jeweilige Abdichten des Duftträgers 10 verzichtet werden.

Nachdem der Luftstrom in der Fig. 6 nun die Einlaßöffnung 40 bzw. die Dichtklappe 43 des Duftträgers 10 passiert hat, gelangt er in ein vorzugsweise spiralförmiges Leitungssystem, die Dufthelix 29, (Fig. 6).

Innerhalb der Dufthelix 29 befindet sich vorzugsweise eine sehr große Anzahl von fächerförmig angeordneten Duftplättchen 26 (Fig. 6), die aus festen, stark porösen Materialien bestehen (z.3. Silikonschwamm o.ä.m.).

In den fächerförmig angeordneten Duftplättchen 26 ist dabei eine große Menge von Duftstoffen vorzugsweise in trockener Form gespeichert, welche beim Hindurchfließen der eingeleiteten Luft an den Luftstrom abgegeben werden.

Um in der Dufthelix 29 einen gleichmäßigen Luftwiderstand zwischen den Duftplättchen und dem durchfließenden Luftstrom zu erreichen, kann es dabei auch zweckmäßig sein, den Rohrdurchmesser in der Helix zum Ende der Spirale hin kontinuierlich zu verjüngen, so daß das Rohr dann sowohl spiralförmig geführt als auch in sich leicht konisch ist.

Dabei sind ebenfalls die verschiedensten Arten der Duftkontaktierung und auch der Duftspeicherung möglich. Ebenso kann der Luftstrom auf mehrere Arten durch den Duftträger oder die Duftrolle und dergleichen geführt werden, z.B. kann-sich die Leitung innerhalb der Duftrolle befinden und wird durch den Duftträger aus der Duftrolle herausgeführt etc.

Durch die Kombination von spiralförmiger Leitungsführung in der Dufthelix 29 (Fig.6) mit der fächerförmigen Aufteilung der Duftplättchen 26 sowie mit der porösen, schaumartigen Oberfläche dieser Duftspeicher wird insgesamt eine extrem große Oberfläche der dufttragenden Teile erreicht.

Hierdurch wird schon beim Hindurchfließen von geringen Luftmengen eine merkliche Abgabe von Duftstoffen erreicht.

Diese intensive Anreicherung mit Duftstoffen ist auch wichtig, da, wie weiter unten beschrieben wird, die Durchmesser der duftführenden Leitungen 37 zu den Zuschauern extrem klein sind.

Die große Oberfläche der duftführenden Teile in der Dufthelix 29 führt in Verbindung mit der trockenen Speicherung der Duftstoffe auch dazu, daß die Materialien in der Dufthelix 29 den Duft viele Male abgeben können, so daß ein Duftträger 10 bzw. eine Duftrolle 9 sehr viele Filmvorführungen lang verwendet werden kann.

Soweit die Duftrollen 9 der Duft-Schaltbox 7 schließlich doch irgendwann verbraucht sind, können sie vom Filmvorführer gegen neue ausgetauscht werden.

Die Einheit Duft-Schaltbox 7 kann dabei auch so ausgeführt sein, daß sie als ganzes vom Leitungssystem 8 und der Duftrollensteuerung 13 abgekoppelt und ausgetauscht wird.

Das Recycling von verbrauchten Duftrollen 9 funktioniert dabei am preiswertesten und vorzugsweise so, daß in speziell hierfür ausgerüsteten Betrieben die verbrauchten Duftträger 10 einfach in flüssige Substanzen der entsprechenden Duftnoten getaucht werden, worauf sich die Duftplättchen 26 der Dufthelix 29 wieder mit den Duftsubstanzen vollsaugen, wo sie dann nach dem Trocknen wieder in konzentrierter Form verbleiben.
Zur Erleichterung des Aufnehmens von neuen Duftstoffen sowie für die Abkürzung des Trockenvorganges können die Duftträger 10 und die Dufthelix 29 dabei auch so gestaltet sein, daß sie in der Mitte auseinanderklappbar sind.

Hierbei teilt sich die Dufthelix 29 dann in eine obere und eine untere Dufthelixhälfte 29a (Fig.6) und ist dadurch gut von außen zugänglich.

Diese Art der Ausführung dürfte auch den Herstellungsprozeß stark in den Kosten herabsetzen und vereinfachen.

Bei entsprechender Verbreitung des neuen Kinosystems sind auch relativ preiswerte Einwegsysteme verschiedenster Art der Duftrollen 9, der Duftträger 10, der Dufthelix 29 oder sonstiger dufttragender Systeme machbar.

Wie stark und vor allem wie schnell nun die Luft aus der Druckluftleitung 8 insgesamt in der Dufthelix 29 mit Duftstoffen angereichert wird, hängt dabei im wesentlichen von der Stellung des Duftstärkereglers 33 und des Luftstromreglers 32 ab.

So ist es beispielsweise möglich, den angestrebten Duft zuerst nur ganz schwach einzuleiten, indem die Regler 32 und 33 den Luftstrom zunächst nur wenig in die Dufthelix 29 einleiten und der Hauptstrom der Luft weiterhin am Duftträger 10 vorbeigeleitet wird (Fig. 6, siehe den gestrichelt darestellten Duftstärkeregler 33).

Eine entprechende Stellung des Duftstärkereglers 33, der hierbei nur wenig öffnet (Fig. 6, gestrichtelte Darstellung), sowie entsprechend des Luftstromreglers 32, wird nun angesteuert, indem zunächst kein Vollimpuls 19 (= volle Duftabgabe), sondern nur ein Teilimpuls 20 (= geringe Duftabgabe) vom durchlaufenden Filmmaterial 12 an den Impulsabtaster 4 abgegeben wird (Fig. 1 und 3).

Der Impulsabtaster 4 gibt daraufhin ein entsprechendes 'kleineres' Signal an die Duftrollensteuerung 13 ab.

Die Duftrollensteuerung ist hierbei mit einer Reglersteuerung 38, welche die Regler 32 und 33 steuert, verbunden und gibt an diese den 'kleinen' Steuerungsbefehl für die Duftstärkeregler 32 und 33 weiter, worauf sich diese dann nur ein entsprechend kleines Stück öffnen.

Hierdurch erfolgt somit zunächst nur eine schwache Duftabgabe der Dufthelix 29 oder entsprechender anderer dufttragender Systeme (Fig. 6, gestrichelte Darstellung).

Durch diese Steuerungsmöglichkeiten lassen sich je nach Stärke der Teilimpulse 20/21 beliebig viele Nuancierungen der Intensität des jeweiligen Duftes erreichen.

Der weitere Öffnungsgrad des Duftstärkereglers 33 und des Luftstromreglers 32 mit entsprechender Duftgradveränderung kann dann durch weitere Teilimpulse 20 oder einen Vollimpuls 19, je nach den Erfordernissen des Filmgeschehens, weitergesteuert werden.

Durch diese Technologie ist es möglich, bestimmte Duftereignisse des Films, die sich gemäß der Handlungslogik eigentlich nur sehr langsam bemerkbar machen können, entsprechend langsam gewissermaßen 'einzublenden' (z.B. ein junges Paar flaniert zur Stadt hinaus und kommt dabei mehr und mehr ins Grüne - zuerst nimmt man nur sehr schwach den Duft von Gräsern und Bäumen wahr und erst allmählich wird der Duft stärker).

Diese Einblend-Technik ist natürlich auch rückwärts möglich, d.h. daß ein Duft ebenfalls nach und nach 'ausgeblendet' werden kann, indem der vollständig geöffnete Duftstärkeregler 33 (ebenso der Luftstromregler 32) sich durch "negative Teilimpulse" 21 erst allmählich schließt.
(Filmbeispiel: Ein Mann geht vom Hafen, mit dem typischen Hafen- und Meergeruch, in Richtung Stadt - der Hafengeruch verliert sich erst allmählich).

Die dritte wichtige Art der kontinuierlichen Duftveränderung ist die "Überblendung". Diese läßt sich duch das Zusammenspiel der Duftrolle 9 mit einer zweiten, in der Duft-Schaltbox 7 vorhandenen Duftrolle 9a erreichen (Fig. 5).

Bei der Überblendung wird, während bereits ein bestimmter Duft wahrnehmbar ist, kurzfristig ein zweiter Duft wahrnehmbar gemacht.
(Filmbeispiel: Während bereits eine Gesellschaft einen leicht holzig und rauchig riechenden Saal bevölkert, flaniert eine vornehme Dame durchs Bild und zieht dabei einen gewissen Parfumduft hinter sich her, was kurzfristig den vorherigen Geruch überdeckt.)

Die Überblendung von zwei verschiedenen Duftnoten wird nun ganz einfach durchgeführt, indem zu der bereits laufenden Dufteinleitung, z.B. von Duftrolle 9, ein in der zweiten Duftrolle 9a gespeicherter Duft ebenfalls in die Druckluftleitung 8 eingeleitet wird (wie in Fig.5 dargestellt, allerdings sind bei der Überblendung die hier nur schematisch angedeuteten Duftstärkeregler 33 und 32 genauso, wie in der Duftrolle 9, ebenfalls bei Duftrolle 9a geöffnet, vgl. Fig. 6).

Bei Bedarf kann der Duft der ersten Duftrolle 9 während der Überblendung durch den Duft der zweiten Duftrolle 9a natürlich auch kurz zurückgenommen werden und anschließend wieder verstärkt werden.

Der Einsatz der zweiten Duftrolle 9a macht es auch zeitlich möglich, zwei Duftnoten unmittelbar hintereinander einzuleiten, was bei dem Einsatz von nur einer Duftrolle wohl nicht machbar sein dürfte.

Bei einem schnellen Duftwechsel mit einer einzigen Duftrolle 9 müßte zuerst der Luftdurchfluß unterbrochen werden, der Duftträger 10 aus der Verbindung mit dem Duftanschluß 25 gelöst und ein neuer Duftträger angesteuert und eingeschaltet werden, und schließlich erneut Luft eingeleitet werden.
Wenn dann der eingeleitete Duft noch etwas Zeit benötigt, um bis zum Zuschauer zu gelangen (siehe weiter unten unter: "Synchronisation"), ist klar, daß eine rasche Duftfolge bei nur einer vorhandenen Duftrolle 9 äußerst schwierig werden dürfte.

Diejenigen Duftnoten, die während des Filmgeschehens also sehr kurz aufeinander folgen, werden somit zwecks schnellem Duftwechsel vorzugsweise alternierend auf den Duftrollen 9 und 9a gespeichert.

Hierdurch kann die bei einer bestimmten Szene eventuell einmal notwendig werdende Schnelligkeit eines Duftwechsels also nahezu beliebig vom Filmregisseur bestimmt werden, da mit der alternierenden Schaltfolge der Duftrollen 9 und 9a das Instrument des schnellen Duftwechsels ebenfalls zur Verfügung steht.

Für ein derartiges, vollkommen neues Medium in der cinematographischen Kunst wird sich mit der Zeit wahrscheinlich auch eine vollkommen neuartige, filmische Ausdrucksweise entwickeln, ähnlich, wie das nach der Einführung des Tonfilms auch der Fall war. Welche Anforderungen daher noch an das neue Medium gestellt werden, ist noch schwer vorhersehbar.
Sollte daher im Laufe des Einsatzes dieses neuen Instrumentariums der Dufteinspielung zu Filmszenen jedoch in bestimmten Fällen, wie eventuell sehr schnell ablaufenden Szenen, ein noch schnellerer Duftwechsel gewünscht werden, könnte man die Duft-Schaltbox 7 auch noch mit einer dritten, nicht dargestellten Duftrolle bestücken.

Auf einer derartigen, dritten Duftrolle könnten u.U. auch besondere Duftnoten, die für Werbungs-Vorfilme oder vorherige Werbung für andere neue Filme mit Filmtrailern benötigt werden, gespeichert werden. Soweit eine dritte Duftrolle sich als grundsätzlich nicht notwendig erweist, werden die Duftnoten von Werbefilmen und Filmtrailern etc. ebenfalls auf einer der Duftrollen 9 oder 9a gespeichert.

Mit dem Einsatz der soeben beschriebenen Techniken des Ein-, Aus- und Überblendens sowie des Schnellwechsels dürften soweit die meisten der denkbaren filmischen Situationen dufttechnisch im wesentlichen beherrschbar sein.

Sofern im gleichen Kinosaal täglich mehrere verschiedene Filme gezeigt werden, wird der Duft für die anderen Filme entspechend durch eine andere Duftrolle oder eine andere Duftschaltbox 7 weitergegeben. Sofern also z.B. nachmittags das Programm 1, abends das Programm 2 und nachts das Programm 3 im gleichen Kinosaal gezeigt werden, stehen hierfür vorteilhafterweise auch drei Duftschaltboxen 7 in der Filmvorführkabine zur Verfügung (nicht dargestellt).

Damit man für die verschiedenen Filme nicht jeweils eine andere Duftschaltbox 7 an den Duftanschluß 25 anschließen muß, sind die drei nebeneinander plazierten Duftschaltboxen 7 dabei auch an drei eigene Duftanschlüsse 25, die alle mit der Druckluftleitung 8 verbunden sind, angeschlossen.

Bei einem Wechsel des Programms wird der Duftanschluß 25 nun einfach vom, Filmvorführer auf den entspechenden Film umgeschaltet. Bei einer Weiterentwicklung des Systems ist es auch möglich, daß jeder Film über ein entsprechendes Duftanschlußsignal sich selbst den zugehörigen Duftanschluß einschaltet.

Das in Fig. 7 dargestellte Ausführungsbeispiel ist besonders für die Vermeidung von Problemen eingerichtet, die eventuell bei der weiter oben beschriebenen Technik der Duftüberblendung eintreten könnten.

Bei der Technik der kurzfristigen Duftüberblendung ist es möglich, daß sich bei häufiger Überblendung innerhalb einer einzigen Leitung 8 (Fig. 5) das Problem der sogenannten "Aromaübertragung" ergibt.

Es könnte dabei vorkommen, daß ein in der zweiten Duftrolle 9a befindlicher, starker Duftstoff, der bei der Überblendung dann zuerst in die erste Duftrolle 9 mit einem schwachen Duft hineingeleitet wird (siehe Fig. 5, aber mit geöffneten Reglern bei beiden Duftrollen) nach mehrmaliger Vorführung des Films den ersten Duft in der Duftrolle 9a vollständig verdrängt hat.

(Beispiel: in einer alten italienischen Villa mit ihrem leichten spezifischen Eigengeruch, der bereits im Kino eingespielt wird, zündet der in die Totale herangeholte Hauptdarsteller im Halbdunkel ein Streichholz an, um mehr zu sehen - der Grundgeruch des Hauses wird kurz durch den dabei entstehenden, typischen Schwefelgeruch überdeckt) - Mögliches Ergebnis:

Nach mehreren Filmvorführungen hat der Duftträger 10 der ersten Filmrolle 9 mit dem entsprechenden, zu mehreren Szenen gehörenden Hausgeruch bereits einen starken Schwefelgeruch aus dem zweiten 'eingeblendeten' Duftträger 10 der Duftrolle 9a angenommen.

Dieser eigentlich nur kurz eingeblendete Streichholzgeruch würde dann bei der nächsten Filmvorführung bereits bei Öffnung der ersten Duftrolle 9 mit dem Hausgeruch wahrnehmbar werden, was beim Zuschauer den fälschlichen Eindruck erweckt, es würde im ganzen Haus brennen.

Um ein derartiges eventuelles Problem zu vermeiden, ist es nun zweckmäßig, die Druckluftleitung 8 des Ausführungsbeispiels in (Fig.7) im Bereich der Duftrollen 9 und 9a zu teilen.

In diesem Fall werden die Dufteinleitungen bei einer Überblendung aus den beiden Duftrollen 9 und 9a innerhalb von getrennten Leitungen durchgeführt, so daß der eigentliche Überblend-Effekt erst nach den Duftrollen in der wieder zusammengeführten Druckluftleitung 8 stattfindet (Fig. 7).

Hierdurch wird eine bei Überblendungen mögliche Übertragung von Duftstoffen einer Duftrolle 9a auf die andere Duftrolle 9 (Fig.5) zuverlässig vermieden (Fig. 7).

Nach einer Analyse von rund 50 bedeutenden Filmwerken hat sich bezüglich des Duftwechsels gezeigt, daß es bei den meisten Filmen nur etwa 20 bis maximal 35 Szenen gibt, die mit insbesondere verschiedenen Duftnoten sinnvoll begleitet werden können.

Darüber hinaus gibt es aber meistens jeweils etwa 5 bis 10 Hauptszenen-Orte, die häufiger wiederkehren und deren Duftnoten sich daher mehrmals wiederholen (z.B. ein bestimmtes Zimmer, eine Wohnung, ein bestimmtes Auto, eine bestimmte Gegend, wie ein Wald etc.).

Von den Hauptszenen-Orten würde man im Durchschnitt vermutlich der Hälfte keinen eigenen Duft zuordnen.
Die andere Hälfte der Hauptszenen-Orte wiederholte sich dabei etwa zwischen 2 und 12 mal, im Durchschnitt etwa 7 mal, so daß sich die entsprechenden Duftnoten ebensooft wiederholen würden.

Aus diesem Grunde läßt sich das ganze Duft-Equipment eines Films erheblich vereinfachen, wenn nicht jeder Duft auf einem eigenen Duftträger 10 in der Duftrolle 9 gespeichert wird, sondern die häufig wiederkehrenden Duftnoten eines Hauptszenen-Ortes jeweils auf einem einzigen oder nur auf zwei Duftträgern 10, die dafür etwas größer ausgeführt werden.

Bei einem derartigen Ausführungsbeispiel werden die z.B. 4 Duftnoten derjenigen Hauptszenen-Orte, denen man einen eigenen Duft zuordnen kann, dann im Durchschnitt z.B. 7 mal aus dem jeweils gleichen Duftträger 10 eingeblendet, wodurch sich also etwa 24 Duftträger einsparen lassen.

Entsprechend läßt sich in den Figuren 3a und 5 erkennen, daß auf einer der beiden Duftrollen 9 oder 9a mehrere größere Duftträger untergebracht sind.
Bei dieser Ausführungsweise werden dann ebenfalls entsprechende Impulse, die die wiederkehrenden Duftträger mehrmals ansteuern, auf dem Filmmaterial angebracht.

Die weiter oben beschriebene, allmähliche graduelle Steigerung der Stärke eines eingeleiteten Duftes über die jeweilige Stellung der Regler 32 und 33 ermöglicht noch eine weitere interessante Anwendung.

Wie bei der Beschreibung der Duftträger 10 bereits erwähnt, wird die Stärke der Duftabgabe durch die Dufthelix 29 nach häufiger Vorführung des Films irgendwann schwächer.

Um nun zu vermeiden, daß das letzte Drittel aller mit einer Duftrolle 9 möglichen Filmvorführungen, bevor die Duftrolle schließlich ganz verbraucht ist, mit unverhältnismäßig schwachen Duftimpulsen begleitet wird, kann man in die Duftrollensteuerung 13, die über die Reglersteuerung 38 auch die Duftstärkeregler 32 und 33 steuert, eine elektronische Verstärkungsschleife einbauen.

Die Verstärkungsschleife wirkt nun einem Verblassen der Duftimpulse entgegen, indem z.B. ab dem letzten Drittel der möglichen Vorführungen die Stärkegrade der Dufteinleitung durch die Regler 32 und 33 kontinuierlich stärker als ursprünglich eingesteuert werden.

In diesem Fall würde der Öffnungsgrad des Duftstärkereglers 33 (und zugleich der Schließungsgrad des Luftstromreglers 32) bei gleichem Signal also jeweils etwas größer ausgesteuert werden als ursprünglich.

Auf diese Weise steht dann einer immer schwächer werdenden Duftabgabe der Dufthelix 29 eine immer stärker werdende Dufteinleitung durch die Regler 32 und 33 gegenüber und gleicht diese kontinuierlich aus.

Hierbei kann ein Vollimpuls 19, der ja schon die volle Duftabgabe beinhaltet, eigentlich nicht mehr verstärkt werden.
Damit daher auch ein Vollimpuls in seiner Intensität erhalten bleibt, werden die Duftträger 10 nun so abgestimmt, daß sie im Normalbetrieb, also noch bevor die Duftabgabe schwächer geworden ist, z.B. schon bei einer 70 % Öffnung des Duftstärkereglers 33 den vollen Duft (entsprechend dem Vollimpuls 19) abgeben.

Hierdurch kann der Duftstärkeregler 33 (entsprechend Regler 32) die Duftstärke dann noch bis zu seiner 100 % Öffnung nachregeln, bis die Duftrolle 9 schließlich ausgetauscht werden muß.

Wie weit die Duftrollensteuerung 13 den Duft nachregelt, kann dabei über ein darin befindliches Zählwerk geregelt werden, welches dann ab der soundsovielten Vorführung die kontinuierlich stärker werdende Verstärkungsschleife einschaltet.

Die Nachsteuerung kann dabei, falls bestimmte Duftträger 10 schneller in ihrer Wirkung nachlassen als andere, sogar duftträgerspezifisch eingestellt werden.

Die entsprechende Information könnte z.B. am Anfang des Films 12, wie auch alle anderen Signale (Fig.3), plaziert und über den Impulsabtaster 4 an die Duftrollensteuerung 13, die dann auch die Duftstärke über die Reglersteuerung 38 und die Regler 33/32 entsprechend individuell nachregelt, weitergegeben werden. Das Zählwerk stellt sich dann bei Einsatz einer neuen Duftrolle 9 wieder auf "Null" zurück.

Nachdem nun der Luftstrom durch die Dufthelix 29 hindurchgeströmt ist und hierbei je nach Gradeinstellung der Regler 32 und 33 mit Duftstoffen angereichert wurde (wir sagen im folgenden "beduftet"), passiert er am Ende der Dufthelix 29 die Dichtklappe 44 und wird wieder in die Druckluftleitung 8 geleitet und durch den nachfolgenden Kompressordruck weitertransportiert.

Die "beduftete" Luft wird nun durch die Druckluftleitung 8 in die Mitte des Kinos zum Hauptverteiler 11 geführt, wo sich die Druckluftleitung in mehrere, z.B. vier Hauptleitungen 35, teilt (Fig. 1).Von dort wird die beduftete Luft zu mehreren Zwischenverteilern 14 in Verteiler-Leitungen 36 geführt und von dort zu den Endverteilern 17 und schließlich über die Einzelleitungen 37 zu den Duftgebern 28 der Armlehnen 27 der einzelnen Kinositze 16 und somit zum Zuschauer (Fig.1). Der genaue strukturelle und zeitliche Ablauf hiervon wird weiter unten im einzelnen beschrieben.

Um die soweit geschilderten Steuerungsabläufe, wie auch die im folgenden noch beschriebenen Funktionen, jederzeit überblicken zu können, kann es u.U. nützlich sein, die ablaufenden Funktionen optisch sichtbar zu machen.

Zu diesem Zweck kann die Duft-Schaltbox 7 mit einem Kontroll-Terminal 39 mit Bildschirm und Tastatur ausgerüstet werden (Fig. 4).

Auf dem Bildschirm eines derartigen Terminals 39 werden nun alle Abläufe der gesamten Duftsteuerung festgehalten. Hierbei wird der laufende Text nebst einer Szenenbezeichnung eingeblendet und neben dem Text markiert, an welcher Stelle welche Signale und Steuerbefehle ablaufen.
Auch-die Einstellung der bereits beschriebenen, auf jeden Duftträger zugeschnittenen Verstärkungsschleife in der Duftrollensteuerung 13 kann hier eingeblendet und überwacht werden.
Sollte aus nicht vorhersehbaren Gründen die Duftrollensteuerung 13 einmal allgemein zu starke oder zu schwache Duftnoten ansteuern und in die Druckluftleitung 8 einleiten, könnte dies über das Terminal 39 nachgeregelt werden.

Für eine derartige Kontrolle kann auch eine einzelne Duftleitung 37 in die Filmvorführerkabine geleitet werden, wo sich der Filmvorführer dann auf Abruf einen Eindruck über den Stärkegrad der Dufteinleitung verschaffen kann.

Eine weitere Möglichkeit der dufttechnischen Kontrolle, welche die zuvor beschriebenen in der Genauigkeit noch übertreffen könnte, ist der Einbau eines nicht dargestellten, sogenannten "Scentanalysers", der ebenfalls an das Kontrollterminal 39 oder direkt an die Duftrollensteuerung 13 angeschlossen wird.

Der Scentanalyser ist zwar bei vertretbaren Kosten nicht in der Lage, einen bestimmten Duft selbst zu spezifizieren, kann aber anhand der Ausgangsdaten für einen Vollimpuls im Luftstrom elektronisch herausmessen, wieviel Prozent des Duftes eines Vollimpulses 19 aktuell in das Verteilernetz eingespeist werden.

Sofern die gemessenen Daten also stark von den vorgegebenen Daten abweichen, kann über entsprechende Nachregelsignale des Scentanalysers entweder über die Duftrollensteuerung 13 (die auch die Duftstärke über die Regler 33 und 32 steuert) oder über das Kontrollterminal 39 die Duftstärke korrigiert werden.

Soweit wurde der Ablauf der einzelnen Funktionen der Dufteinleitung bei der Vorführung von Kinofilmen also in seinem Schema dargestellt.

Um mit den soweit dargestellten Techniken jedoch ein vollendetes filmisches Gesamtkunstwerk herstellen zu können, sollte die szenenbezogene Dufteinleitung für Kinofilme auf dem Weg vom Projektor zum Zuschauer möglichst noch die folgenden Bedingungen erfüllen:
1.) Schnelle Duftübertragung verbunden mit extremer Verringerung des Luftvolumens (Schnelligkeit und Minimalisierung).
2.) Der Duft muß genau gleichzeitig mit der zugehörigen, auf der Leinwand vorgeführten Szene für den Zuschauer wahrnehmbar werden können (Szenen-Synchronisation).
3.) Der Duft muß die Bedingung 2. nicht nur für einen Zuschauer, sondern für alle Zuschauer im gesamten Kino gleichzeitig erfüllen (Gleichzeitige Wahrnehmbarkeit).

Zu 1.) Schnelligkeit und Minimalisierung: Das Mikro-Luftsystem.

Für die Durchführung des gesamten Ablaufes der szenengenauen Duftzuführung ist es sehr wichtig, daß der Duft schnell und präzise vom Duftträger 10 zum Kinositz 16 bzw. zum Zuschauer transportiert wird.

Hierfür ist es wiederum sehr wesentlich , die Luftmenge , die insgesamt benötigt wird , um den Duft vom Duftträger 10 zum Kinositz 16 zu transportieren , möglichst gering zu halten , da sonst wahrscheinlich gravierende Probleme auftauchen würden, wie zum Beispiel unangenehme Überlagerungen einzelner Düfte im Vorführraum des Kinos , eine starke Geräuschentwicklung durch große, bewegte Luftmassen oder eine den Zuschauer störende Luftzirkulation , abgesehen von dem hierzu nötigen gigantischen Kompressor.

Um die beim Zuschauer insgesamt austretende Luftmenge zu minimalisieren , wird zunächst die Leitungsinnenfläche der Einzelleitungen 37 , die zu den einzelnen Kinositzen führen ( sowie entsprechend aller weiteren Leitungen) , extrem klein gehalten In dem in Fig.8 dargestellten Ausführungsbeispiel ist eine entsprechend miniaturisierte Einzelleitung 37 in der vorzugsweisen Originalgröße dargestellt .

Zugleich wird die herangeführte Luft im Duftträger 10 bzw. in der Dufthelix 29 insgesamt verhältnismäßig intensiv mit Duftstoffen angereichert , damit schon bei einer sehr kleinen , zugeführten Luftmenge der gewünschte Duft bemerkbar wird.

Im Verhältnis zu bestimmten Klimaanlagen , wo in großen Sälen die klimatisierte Luft in großen Röhren teilweise auch zu einzelnen Sitzplätzen geführt wird , ist die Leitungsinnenfläche der hier verwendeten Einzelleitungen 37 extrem miniaturisiert, und zwar z.B. auf etwa ein Tausendstel der Leitungsinnenfläche von Einzelzuführungen einer derartigen Anlage, d.h. also auf etwa 8 - 12mm² Fläche bzw. 3 - 4mm Innendurchmesser.

Entsprechend gering sind die Luftmengen , die mit einem derartigen Mikro-Luftsystem verteilt werden. Pro Sekunde wird hierbei vorzugsweise weniger als 1 Liter Luft abgegeben , bzw. nur zwischen 0,3 und 0,005 Liter/Sek .

Je nachdem , wie intensiv die verwendeten Luftmengen mit Duftstoffen versehen werden können , ist es bei anderen , nicht dargestellten Ausführungsbeispielen auch möglich , die Leitungsinnenfläche auf weniger als den zehntausendsten Teil von üblichen Luftverteilersystemen zu reduzieren . Bei Leitungsinnenflächen von weniger als 2,5 mm² werden hierbei nur noch Luftmengen zwischen etwa 0,00001 und 0,005 Liter/ Sek. abgegeben.

Will man Duftnoten mit normalen Luftverteilungssystemen , z.B . einer Klimaanlage, verteilen, wie dies bereits mehrfach erfolglos versucht wurde , muß man dafür die rund 1.000 bis 10.000fache Luftmenge zu jedem einzelnen Zuschauer hinpumpen.

Die großen Probleme, welche eine derartige Verteilungsart als Basis für ein Duftsystem für Unterhaltungsmedien mit sich bringt, wie die allgemeine Schwierigkeit, derartige Luftmengen technisch präzise zu beherrschen, Überlagerungen von mehreren Düften im Vorführraum, sowie ein außerordentlicher finanzieller Aufwand durch normale , d.h. also im Verhältnis hierzu enorm große Verteilungs- und Luftabsaugsysteme, läßt sich mit einem Mikrosystem perfekt vermeiden .

Mit der Minimalisierung der transportierten Luftmengen und der gleichzeitigen Intensivierung der Dufteinspeisung läßt sich somit das 1 . Problem des schnellen Transportes und der hochpräzisen Verteilung zum einzelnen Zuschauer bewältigen.
Noch nicht vollständig gelöst ist damit die Notwendigkeit, daß die eingeleiteten Duftnoten auch zeitlich genau zur Szene passend abgegeben werden.

Zu 2.) Szenen-Synchronisation.
Die in den meisten Fällen vorteilhafterweise synchrone bzw. szenengenaue " Einspielung " der einzelnen Düfte läßt sich auf mehrere Arten erreichen :
A. ) Der Duftimpuls wird auf dem laufenden Film nicht gleichzeitig mit der betreffenden Szene , sondern einige Sekunden früher abgegeben, und zwar so weit vorverlegt , wie ein Duftimpuls auf dem Film bis zur Umsetzung beim Zuschauer benötigt.

Dauert es also 5 Sekunden , bis ein auf dem Film abgegebenes Duftsignal umgesetzt , bis zum Zuschauer transportiert und von diesem wahrnehmbar wird , wird der entsprechende Impuls 18-21 (Fig . 3) während der Filmvorführung, also 5 Sekunden vor der Szene von dem laufenden Filmmaterial 12, an den Impulsabtaster 4 innerhalb des Filmprojektors 1 (Fig.1) abgegeben.

Im vorliegenden Ausführungsbeispiel liest der Impulsabtaster 4 zunächst den etwas weiter vorverlegten Impuls "Pre-Code" 18 und aktiviert dadurch die Duftrollensteuerung 13. Hierdurch steuert die Duftrollensteuerung 13 den Duftträger 10 auf der Duftrolle 9 oder 9a an, wodurch dieser vor dem Duftanschluß 25 positioniert wird (Fig .4 und 5).

Durch die weiteren , etwas weniger vorverlegten Impulse 19-21 wird nun der Öffnungsgrad des Duftstärkereglers 33 und des Luftstromreglers 32 (Fig. 6 und 5) eingesteuert , um die Einleitung des szenischen Duftes zu erreichen, und zwar jetzt so weit vorverlegt, wie der Weg vom geöffneten Duftträger 10 noch bis zum Duftgeber 28 am Kinositz 16 (Fig. 1 und 11) bzw. zum Zuschauer benötigt.

Da jedoch bei verschieden großen Kinos der Weg vom Projektor 1 bzw. von der Duftschaltbox 7 bis zum Zuschauer verschieden lang sein kann ( Fig. 1) , sollten die Impulse auf dem Filmmaterial 12 so weit vorverlegt werden , daß der Vorlauf für eine maximal denkbare Kinogröße ausreichend ist.

Bei kleineren Kinos mit kürzerem und schnellerem Weg wird der Signalvorlauf dann , damit der szenengebundene Duft nicht zu früh wahrnehmbar wird , in dem Impulsabtaster 4 oder in der Duftrollensteuerung 13 entsprechend elektronisch verzögert . Wenn also ein Signal für die maximale Kinogröße 6 Sekunden vorverlegt auf dem Filmmaterial 12 erscheint , wird es für ein kleineres Kino, welches z.B. nur vier Sekunden Vorlauf benötigt, erst zwei Sekunden später vom Impulsabtaster 4 an die Duftrollensteuerung 13 weitergegeben .

Zur Erreichung der gleichen Wirkung kann auch die Durchflußgeschwindigkeit der kürzeren Leitungen kleinerer Kinos über Durchmesseränderungen oder eine langsamere Pumpgeschwindigkeit des Kompressors 6 oder andere Verlangsamungsmechanismen entsprechend angepasst werden .

Ein weiteres Problem kann entstehen , wenn nach einem Filmriss das Stück des Films 12 fehlt , welches den "Pre-Code" 18 zur Ansteuerung des Duftträgers 10 enthält ( Fig . 3). In diesem Fall würden die nachfolgenden Duftimpulse 19-21 dann auf einen falschen Pre-Code 18 bezogen .
Aus diesem Grunde wird jeder Pre-code 18 jeweils zweimal signalisiert, einmal zur echten Vorlaufzeit und ein zweites mal unmittelbar vor dem zugehörigen Impuls .

So wird der szenische Duft nach einer durch Filmriss fehlenden Stelle zwar leicht verzögert übermittelt , aber immer noch richtig zur zugehörigen Szene zugeordnet.

Da aber bei einem fehlenden Filmstück auch die Duftimpulse 19-21 abhanden kommen können und dann die Reihenfolge insgesamt durcheinander kommen könnte , werden alle Impulse auf dem Film zusätzlich mit einer für den Impulsabtaster 4 ( Fig. 1) lesbaren "numerischen Kennung" 22 versehen (Fig. 3).

Diese fortlaufend numerierten Kennungen 22 besitzen dabei jeweils die gleiche Kennungszahl wie der für diese Szene zugehörige und entsprechend kodierte Duftträger 10 auf der Duftrolle 9 oder 9a (Fig. 4 und 5) .

Die Kodierung der Duftträger kann u.U. auch entfallen , wenn der jeweilige Duftträger 10 durch die Duftrollensteuerung 13 allein durch seine Stellung innerhalb der Duftrolle 9 lokalisiert wird .

Als Orientierung dient der Duftrollensteuerung 13 dabei eine dem Filmanfang zugeordnete "0-Stelle" 46 auf der Duftrolle 9 (Fig .4) .

Vor der Weitergabe jeglicher Duftsignale wird nun von dem Impulsabtaster 4 oder von der Duftrollensteuerung 13 zuerst verglichen, ob es sich um den richtigen Duftträger 10 auf der Duftrolle 9 oder 9a handelt, und bei abweichenden Kennungen werden dann entsprechend viele Duftträger 10 übersprungen und sofort der richtige Duftträger 10 eingeschleust (Fig.4 und 5) .

Auf diese Weise läuft bei fehlenden Impulsen zwar zunächst eine Szene ohne die vorgesehene Dufteinspielung ab , aber bei der nächsten Szene wird dann Dank der numerischen Kennung der "falsche" Duft der vorherigen Szene übersprungen und wieder der richtige , szenisch zugehörige Duft eingespielt.

Bezüglich des weiteren Transportes der bedufteten Luft zum Zuschauer sind mehrere Betriebsarten der Druckluftleitung 8 mit ihren Verzweigungen in die Hauptleitungen 35 , Verteiler-Leitungen 36 und Einzelleitungen 37 möglich.
Im vorliegenden Ausführungsbeispiel erzeugt der Elektrokompressor 6 nur einen sehr geringen Überdruck , der gerade dazu ausreicht , um sich bis zum Luftaustritt an den Duftgebern der Armlehnen 27 der einzelnen Kinositze 16 hin fortzusetzen.

Hierbei bleibt ein steter Luftstrom zwischen Elektrokompressor und Kinositz 16 erhalten , der aber , wie weiter oben beschrieben, durch die sehr klein gehaltenen Einzelleitungen 37 im Umfang sehr gering ist und vom Zuschauer außer bei Dufteinspeisungen nahezu nicht bemerkt wird .

B . ) Bei einem weiteren , nicht dargestellten Ausführungsbeispiel werden die einzelnen Duftsteuerimpulse ebenfalls einige Zeit vor der jeweiligen Szene vom durchlaufenden Filmmaterial abgegeben , worauf die Duftstoffe ebenfalls in die Druckluftleitung 8 und dann Richtung Zuschauer geleitet werden .

In diesem Fall wird aber der Luftstrom bei der letzten Verteilereinrichtung , Endverteiler 17, durch ein nicht dargestelltes Verteiler-Ventil 41 gestoppt , sobald die beduftete Luft dort ankommt. Hierdurch baut sich in der Gesamtleitung ein gewisser Überdruck auf , im vorliegenden Fall sind es z.B. 0,6 Atü.

Der richtige Schließzeitpunkt des Verteiler-Ventils 41 am Endverteiler 17 wird dabei beispielsweise über ein vorgegebenes Zeitintervall eingegeben , welches der Fließzeit des Duftstroms vom Duftträger 10 zum Endverteiler 17 entspricht und welches bei der Montage der Leitungen ermittelt wird (siehe auch Fig. 1) .

Dauert es also z.B. 3 Sekunden , bis der Duft vom Duftträger 10 bis zu den Verteiler-Ventilen 41 fließt , sendet der Impulsabtaster 4 (oder alternativ die Duftrollensteuerung 13) also immer 3 Sekunden , nachdem ein Duft eingegeben wurde , über eine direkt zu den Verteiler-Ventilen 41 reichende Signalleitung ein Schließsignal an die Verteiler-Ventile 41 .

Eine andere Möglichkeit , den richtigen Schließzeitpunkt der Verteiler-Ventile 41 zu ermitteln , wäre beispielsweise ein luftmengengesteuerter Verschluß , der sich nach Durchfluß der Luftmenge, die vom Duftträger 10 bis zum Verteiler-Ventil 41 benötigt wird, verschließt.

In diesem Ausführungsbeispiel wird der szenische Duft also , lange bevor die Szene erscheint , bis kurz vor den Kinositz 16 zum Endverteiler 17 geführt und dort kurzfristig " gespeichert ".

Erst unmittelbar bevor die betreffende Szene nun erscheint , öffnen sich die Ventile in den Endverteilern 17 durch einen speziellen , auf dem Filmmaterial aufgebrachten Ventil-Impuls 23 (Fig. 3) , der vom Impulsabtaster 4 über die direkt zu den Verteiler-Ventilen 41 an den Endverteilern 17 reichende Leitungen weitergegeben wird.

Unmittelbar darauf strömt die beduftete Luft das letzte kurze Stück bis zum Zuschauer weiter.

Solange in diesem Ausführungsbeispiel keine Duftzuführung zum Zuschauer vorgesehen ist , findet also, im Unterschied zum vorhergehenden Beispiel, auch kein Luftaustritt am Kinositz statt.

Voraussetzung für diese Technologie ist , daß die Ventile durch entsprechende Ventildämpfungen so leise arbeitend gefertigt werden , daß während der Filmvorführung keine Störgeräusche durch ihre Funktion im Vorführraum entstehen können.

C.) Bei einem weiteren , ebenfalls nicht dargestellten Ausführungsbeispiel, welches eine sehr zweckmäßige Mischform zwischenden beiden vorhergehenden Beispielen darstellt , werden die Duftimpulse 18-21 zeitlich ebenfalls vor der Szene abgegeben.

In diesem Fall leitet der Kompressor 6 die beduftete Luft ebenfalls bis kurz vor die einzelnen Kinositze 16, allerdings ohne dabei einen Überdruck in der Leitung aufzubauen.

Daraufhin wird durch ein Kompressorventil 63 an der Druckluftleitung 8 ( Fig . 5 und 7 ) die weitere Luftzufuhr aus der Druckluftleitung 8 heraus gelenkt oder abgeriegelt .

Durch Abschalten der weiteren Luftzufuhr in der Druckluftleitung 8 bleibt die beduftete Luft nun auch in allen weiteren Verzweigungen der Leitung und somit wenige Meter vor dem Austritt an den Sitzen auch in den Einzelleitungen 37 stehen .

Ein selbsttätiges , vorzeitiges Ausbreiten des Duftes zu den Sitzen 16 hin kann dabei durch den extrem kleinen Leitungsdurchmesser der Einzelleitungen 37 (in Fig.8 in Originalgröße dargestellt) sowie durch den sehr kurzen Zeitpunkt des Zurückhaltens in der Leitung mit Sicherheit ausgeschlossen werden.

Der richtige Schließzeitpunkt des Kompressorventils 63 ( Fig . 5 oder 7) wird hier , wie auch im letzten Ausführungsbeispiel , ebenfalls über ein einprogrammiertes Zeitintervall oder einen Fließmengen-Schalter erreicht.

Erst unmittelbar vor der Szene wird der Luftdruck des Kompressors 6 durch ein entsprechendes geeignetes Signal , z.B. das Ventilsignal 23 , welches hier aber zu dem Kompressorventil 63 führt wieder in die Druckluftleitung 8 eingeleitet , so daß der Luftfluß sich erneut zu den Armlehren 27 der Sitze 16 hin fortsetzt und der entsprechende Duft ausströmt (Fig. 1) .

D.) Bei einem weiterer, in Fig .2 dargestellten Ausführungsbeispiel wird zunächst die Länge der Strecke zwischen Duftträger 10 bzw. Duft-Schaltbox 7 und den einzelnen Kinositzen 16 stark verringert, indem die Duft-Schaltbox 7, nicht wie in Fig . 1, in die Filmvorführerkabine eingebaut wird , sondern in die Mitte des Kinos an die Stelle des bisherigen Hauptverteilers 11 , der damit zur Zentral-Duftschaltbox lla wird ( Fig . 2) .

Entsprechend verlaufen die Druckluftleitung 8a und die Signalleitung 5a nun auch direkt in die Mitte des Vorführraumes zur Zentral-Duftschaltbox 11a ( Fig . 2) .

Hierdurch wird die Strecke bis zu den Kinositzen 16 u.U. bereits so stark verkürzt , daß auf den Signalvorlauf der Duft-Impulse 18-21 eventuell schon verzichtet werden kann , so daß die Impulse 18-21 erst unmittelbar vor der zugehörigen Szene vom ablaufenden Filmmaterial abgegeben werden müssen .

E. ) In einer weiteren , nicht dargestellten Verkürzungsstufe wird die Duft-Schaltbox 7 noch näher zu den Kinositzen 16 verlegt, und zwar an die Stelle der bisherigen Zwischenverteiler 14 , so daß hieraus z.B. die vier zeitgleich arbeitenden Duft-Schaltboxen 14a werden , wobei die entsprechenden Druckluftleitungen und Signalleitungen ebenfalls zu diesen vier Duft-Schaltboxen 14a geleitet werden.

Vorraussetzung für die Verlegungen der Duft-Schaltboxen 11a/14a in den letzten beiden Ausführungsbeispielen zum Zuschauerraum ist natürlich , daß die Lufteinleitungs-Vorgänge in den Duftträger 10 so leise innerhalb des Zuschamerraums bewerkstelligt werden , daß keine Störungen des Filmerlebnisses dadurch hervorgerufen werden können.

F . ) Bei einem weiteren , nicht dargestellten Ausführungsbeispiel wird zwar zu allen Kinositzen 16 ebenfalls ein Verteilersystem (Teile 8 , 35 , 36 , 37 , wie in Fig . 1 oder 2) verlegt , aber im Unterschied zu allen vorherigen Ausführungsbeispielen werden bis zum Erreichen des Kinositzes 16 keinerlei Dufteinleitungen in das Verteiler-System vorgenommen.

Erst am Kinositz 16 wird dann ein Duftprogramm aus einem sehr kleinen Einleitungssystem , welches mit dem Verteilernetz bzw. mit einer Einzelleitung 37 verbunden ist , eingespeist .
Als Speicher für Duftprogramme sind viele Möglichkeiten denkbar , z.B. die weiter unten beschriebenen "Duftdisketten" ( Fig . 17) und dergleichen mehr.
In den weiter oben beschriebenen Möglichkeiten der Duftein- , -aus- und -überblendung ist dieses letztgenannte System aber bei vertretbarem Kostenaufwand bei weitem nicht so anpassungsfähig und vielfältig und daher für die Zielsetzung eines perfekten Illusionskinos als Gesamtkunstwerk nur eingeschränkt anwendbar.

Ferner sind weitere Mischverfahren zwischen den dargestellten Möglichkeiten denkbar.

Zu 3. ) Gleichzeitige Wahrnehmbarkeit.

Um den dritten Teil der Aufgabe , den präzisen Austritt der bedufteten Luft bei allen Kinositzen 16 gleichzeitig zu ermöglichen , werden in einem nicht dargestellten Ausführungsbeispiel die Leitungen zu allen Sitzen alle gleich lang gestaltet .
Soweit dabei die Leitungsentfernungen zu bestimmten Sitzen kürzer als andere sind , wird die fehlende Strecke durch Leer- bzw. Ausgleichswicklungen an den kurzen Leitungen genau so angepasst, daß der Luftaustritt hierdurch genau gleichzeitig und gleich stark stattfindet.

Bei einem weiteren , nicht dargestellten Ausführungsbeispiel werden die Durchmesser der einzelnen Leitungen so aufeinander abgestimmt , daß die Luft bei den Duftarmlehnen 27 aller Kinositze 16 gleichzeitig austritt und wahrnehmbar wird .

Bei einem weiteren , nicht dargestellten Ausführungsbeispiel wird die Gleichzeitigkeit schließlich über eine besondere Ventilführung im Leitungssystem erreicht . Hierbei wird die beduftete Luft, wie schon in den Verfahren zur "Synchronisation" B und C dargestellt , ebenfalls vor der zugehörigen Szene zunächst bis zu den Endverteilern 17 geleitet. Dort wird der Duft dann ebenfalls gespeichert , indem ein luftmengen- oder zeitintervallgesteuertes Verteiler-Ventil 41 die beduftete Luft stoppt , sobald sie das Ventil erreicht .

Kurz bevor nun die zugehörige Szene auf der Leinwand erscheint , wird nun ebenfalls ein Ventil-Impuls 23 (Fig. 3) an die Verteiler-Ventile 41 geschickt.

Die Öffnungszeitpunkte dieser Ventile laufen dann aber nicht gleichzeitig ab.

In diesem Fall werden die Öffnungszeitpunkte der Verteiler-Ventile 41, an deren Leitungen 37 die eingeleiteten Duftnoten etwas zu früh austreten , bereits beim Einbau des Duftleitungssystems geringfügig nachverlegt, und zwar genau so , daß die beduftete Luft aus allen Einzelleitungen 37 schließlich gleichzeitig austritt.

Weitere Möglichkeiten , eine Gleichzeitigkeit der Duftereignisse während der Filmvorführung herzustellen , bestehen in den verschiedensten Mischformen zwischen den einzelnen vorgestellten Verfahren .

Durch die dargestellten Techniken werden insgesamt also die drei vorteilhafterweise erfüllten Anforderungen :
1.) "Schnelligkeit und Minimalisierung" der Duftübermittlung ,
2.) "Szenen-Synchronisation" , also die szenengenaue Duftübermittlung, und
3.) die "Gleichzeitige Wahrnehmbarkeit" der Duftübermittlung
bei allen Kinobesuchern weitestgehend gelöst.

Um das Dufterlebnis jedoch auch für jeden einzelnen Zuschauer perfekt zu gestalten , ist eine individuelle Einstellbarkeit der wahrnehmbaren Duftnoten wahrscheinlich empfehlenswert und mit sehr geringem Aufwand auch machbar.

Die Wahrnehmung von Gerüchen und Düften ist im Verhältnis zur Rezeption von Bildern , Tönen und Musik eine Sache , die individuell stärker verschieden aufgenommem wird.

Aus diesem Grunde ist es u.U. wichtig , daß der einzelne Kinobesucher die Intensität der abgegebenen Duftnoten individuell regeln und notfalls auch ganz abstellen kann .

Um die Stärke der Duftnoten entsprechend individuell einstellen zu können , befindet sich daher z.B. an der rechten Armlehne 27 jedes Kinositzes 16 ein sogenannter Duftdimmer 70 (Fig. 11) , mit dem die Zuschauer mit einer empfindlichen Nase das Dufterlebnis schwächer oder auch ganz abstellen können.

Der Duftdimmer 70 ist dabei direkt mit einem ebenfalls an der Armlehne 27 befindlichen , aber für den Zuschauer nicht sichtbaren Sitzplatz-Duftregler 72 verbunden (in Fig. 9 und 10 etwa in Originalgröße dargestellt).

Sofern man den Duftdimmer 70 nun kleiner stellt , wird in dem Sitzplatz-Duftregler 72 ( Fig.9) ein entsprechender Teil des zugeleiteten Duftes , bevor er am Kinositz austreten kann , über einen kleinen Leitungsregler 61 direkt in eine Rückleitung 56 umgeleitet (Fig. 9 oder 10 bei ganz geschlossenenem Duftdimmer).

Zur Bereitstellung dieser Möglichkeit wird nun zu jedem Sitz nicht eine einfache Zuleitung 37 verlegt, sondern eine Doppelleitung 45 , welche in der Fig. 8 zur Veranschaulichung der Größenverhältnisse etwa in Originalgröße dargestellt ist.

Bezüglich der Montagekosten dieser Leitungen dürfte es praktisch nicht ins Gewicht fallen , ob bei der Montage nun eine Einzelleitung 37 oder eine Doppelleitung 45 verlegt wird.

Bei den wahrscheinlich ohnehin sehr geringen Kosten des Leitungsmaterials (vorzugsweise sehr preiswerte, geruchsresistente Kunststoffleitungen) dürfte eine Doppelleitung 45 kostenmäßig auch sonst kaum Unterschiede zu der äußerst preiswerten Einzelleitung 37 haben. Insofern wäre hierbei mit sehr geringem Mehraufwand bereits eine individuell anpassungsfähige Duftbegleitung von Kinoszenen gewährleistet.

Darüber hinaus kann die Rückleitung 56 der Doppelleitung 45 auch gleichzeitig für eine zweite Funktion verwendet werden.

Soweit die Duftzuleitungen 37 zu den Sitzen täglich für mehrere Kinoaufführungen benutzt werden , könnte es im Laufe der Zeit sein , daß Teile der Rohrleitungen bestimmte Gerüche, die z.B. sehr oft vorkommen , teilweise annehmen.
Auch wenn die Leitungen so beschaffen sind , daß sie keine Gerüche aufnehmen können , ist es denkbar , daß sich in den Leitungen Rückstände bilden oder Schwitzwasser , welches dann ebenfalls unerwünschte Gerüche produzieren könnte.

Unter Zuhilfenahme der bereits zu den Sitzen verlegten Rückleitung 56 kann nun z.B. einmal im Monat sehr einfach eine Reinigung der Duftleitung 37 durchgeführt werden , indem der an den Sitzen befindliche Duftdimmer 70 auf "Null" gestellt wird, wobei sich der Sitzplatz-Duftregler 72 vollständig verschließt (Fig. 10).

Wird nun statt Luft zuerst eine Reinigungsflüssigkeit und anschließend warme Trockenluft durch die Zuleitung 37 geführt, fließen diese über die Rückleitung 56 wieder zur Pumpeinrichtung zurück, wobei mögliche Verunreinigungen dann beseitigt werden.

Statt einer Pumpeinrichtung kann diese Aufgabe auch vom bereits installierten Elektrokompressor 6 erfüllt werden, wenn die Druckluftleitung 8 nach dem Kompressor in ein Zuführgerät geleitet wird, welches die Reinigungsflüssigkeit dann zuführt und auch wieder aus der Rückleitung 56 aufnimmt.

Somit können also auch eventuell auftretende Verunreinigungen der Leitung durch den langfristigen Dauerbetrieb leicht wieder entfernt werden.

Die sehr kleine und preiswerte Doppelleitung 45 ( Fig . 8), bestehend aus der Einzelleitung 37 und der Rückleitung 56, kann dabei also bereis die drei wesentlichsten Funktionen des Duftleitungssystems übernehmen :

a.) die Zuführung der szenengenauen Duftnoten , b . ) die Ableitung von individuell als zu stark empfundenen Dufteinleitungen und c. ) bei 3edarf , die Reinigung der Leitungen.

Für eine neue und faszinierende Technologie der duftmäßigen Begleitung von Filmszenen ist damit also bereits ein sehr preiswertes Instrumentarium für den entsprechenden Umbau von Kinovorführräumen bereitgestellt .

Während bei den Filmvorführungen nun die einzelnen Duftnoten sowohl präzise und szenengerecht als auch auf die individuelle Vorstellung des Zuschauers angepasst ankommen , ist es u.U. auch notwendig , diese Duftnoten nicht zu lange im Raum schweben zu lassen , um eine szenische Überschneidung sowie eine Überlagerung mehrerer Duftnoten zu vermeiden.
Um eine derartige Überlagerung zu verhindern, ist es z.B. möglich , die szenischen Duftnoten jeweils z.B. 3-5 Grad über der Temperatur im Vorführraum des Kinos an den Armlehnen 27 der Kinositze 16, d.h. am Duftgeber 28, abzugeben (Fig. 11) . Hierdurch steigt die mit Duftnoten angereicherte Luft nach oben , was durch eine im Deckenbereich des Kinos befindliche Absauganlage einer üblichen Klimaanlage noch unterstützt werden könnte.
Eine technisch noch wirksamere Möglichkeit, die abgegebenen Duftnoten möglichst kurz im Vorführraum verweilen zu lassen , ergibt sich, wenn den szenischen Duftnoten bestimmte Mischungen von leichtflüchtigen Elementen beigefügt werden, wie z.B. Helium , Argon oder Xenon . Hierdurch bekommt die austretende, sehr kleine Luftmenge ein wesentlich geringeres spezifisches Gewicht als die Umgebungsluft, was zu einem sehr raschen Abzug der abgegebenen Duftbeigaben führt . Diese Aufwärtsbewegung der Luft kann auch mit einem Versetzen der kleinen austretenden Luftmenge in eine Drehbewegung (Prinzip der Windhose) durch eine geeignete, wendelartige Führung der Luft herbeigeführt oder unterstützt werden .

Die vorgeschlagenen Mischungen, z.B. von Helium und Atemluft, sind dabei ohne gesundheitliches Risiko möglich , da entsprechende Mischungen bereits seit Jahrzehnten erfolgreich in der Tauchtechnik Verwendung finden , um Tauchern das Atmen unter Wasser zu erleichtern .

Eine andere Möglichkeit , die Duftnoten nicht zu lange im Bereich der Kinositze verweilen zu lassen , ergibt sich , wenn vorzugsweise am oberen Rand der Sitzrückenlehnen oder sonstigen geeigneten Stellen im Bereich der Kinositze sogenannte Duftresorber 15 eingebaut werden (Fig .11). In den Duftresorbern 15 wird dabei der überflüssige Duft abgesaugt, so daß dieser nicht länger wahrnehmbar ist .

Zwischen den Duftgebern 28 und dem Duftresorber 15 kann sich bei diesem Ausführungsbeispiel ein steter , aber sehr schwacher Luftstrom entwickeln , welcher in Fig . 11 bei einem der Kinositze dargestellt ist . Dieser schwache Luftstrom kann wegen der dabei bewegten , äußerst geringen Luftmengen keine Reizungen der Augen und Nase etc. hervorrufen.

Er bewirkt aber u.U., daß die Wahrnehmung der einzelnen , zu den Kinoszenen eingespielten Duftnoten weitgehend unabhängig davon verlaufen , ob der Kinobesucher den Kopf gerade nach vorn oder nach hinten gebeugt hat , so daß die Duftwahrnehmung in allen möglichen Sitzpositionen gleichmäßig und gleichstark ist .

Die Duftresorber 15 sind dabei an eine Resorberleitung 58 angeschlossen, die in ihren Dimensionen vorzugsweise wesentlich größer ausfällt als die Einzelleitungen 37 (Fig. 13,14 und 8).

Bei einem eventuellen Einbau einer Resorberleitung 58 werden die Leitungen so gestaltet , daß zur Vereinfachung der Verlegearbeiten die Einzelleitung 37 und die Rückleitung 56 mit der Resorberleitung 58 eine Einheit bilden ( Fig . 14) oder aber als Doppelleitung 45 (Fig. 8) mit diesen fest über eine Haltenut 59 verbunden werden können (Fig. 13).

Sobald sich das neue und faszinierende Instrument der duftbegleiteten Filmvorführung weit in der Welt der Unterhaltung ausgebreitet hat , könnte es durchaus sein , daß das Bedürfnis , diese Möglichkeiten manchmal auch außerhalb des Kinos zu nutzen , steigt.

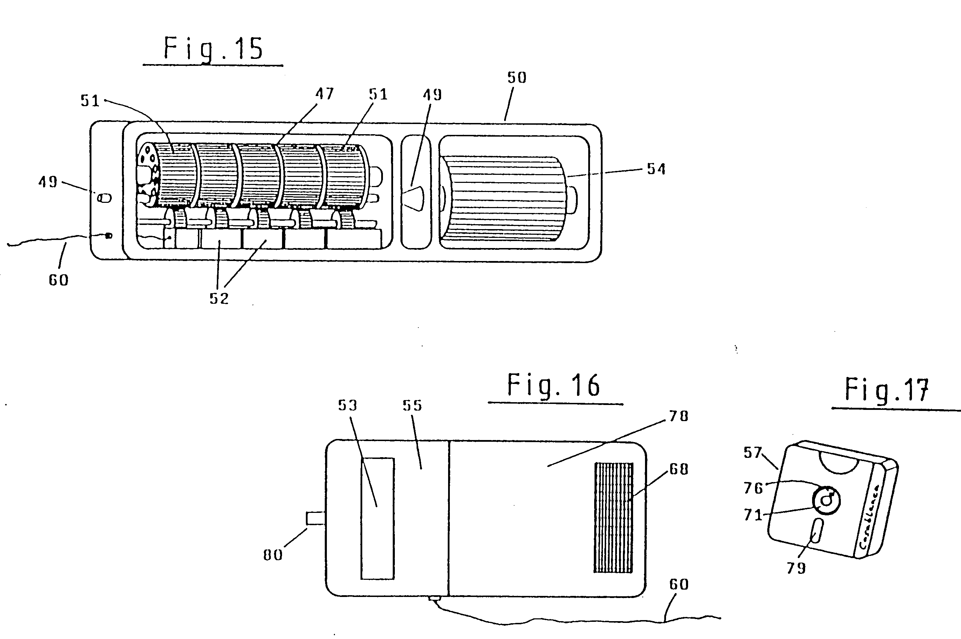

Aus diesem Grunde könnten eines Tages dezentrale Verwendungsarten ebenfalls interessant werden , weshalb die folgenden beiden Anwendungsbeispiele besonders hierauf zugeschnitten sind (Figuren 15-17).

Ein weiteres Ausführungsbeispiel der Erfindung , der sogenannte Duftcomposer 50 , welcher in Fig . 15 dargestellt wird , ist besonders für die Verwendung in kleineren Räumen , für dezentrale Anwendungen und für weniger aufwendige Duftdarbietungen geeignet . Der Duftcomposer 50 kann daher auch als begleitende Darbietung für Musikaufführungen , bei Diavorträgen oder für die Ausstrahlung von Video- und Fernsehsendungen eingesetzt werden .

Der Duftcomposer 50 arbeitet dabei nach einem weitgehend anderen Prinzip als die vorhergehenden Ausführungsbeispiele.

In diesem Fall sind die Duftnoten , die schließlich Verwendung finden , nicht schon fertiggestellt , wie in den Duftrollen 9 und 9a der Ausführungsbeispiele in Fig. 4 , 5 und 7 . Vielmehr werden hier alle benötigten Duftnoten durch das Hintereinanderschalten von mehreren Duftmischrollen 51 erst kurz vor ihrer Verwendung hergestellt ( Fig 15).

In dem Duftcomposer 50 sind dabei z.B. 5 Duftmischrollen 51 hintereinandergeschaltet , welche jeweils z.B. 12 DuftGrundkomponenten enthalten . Hierbei können sehr häufig verwendete Grundkomponenten auch mehrmals in einzelnen Duftmischrollen vorkommen . Auf diese Weise lassen sich mit 60 Grundkomponenten bereits rund 250.000 verschiedene Duftnoten bzw. 25.000 verschiedene Duftnoten in 10 verschiedenen Stärkegraden herstellen .

Mit einem kleinen , innerhalb des Duftkomposers 50 untergebrachten Tangentialgebläse 54 , welches etwas niedertouriger als sonstige Gebläse dieser ohnehin leisen Gebläseart und daher extrem leise läuft , wird nun über die Zuleitung 49 Luft durch die jeweils innerhalb der Zuleitungslinie liegenden Duftgrundkomponenten geführt .

Die mit der Zuleitung 49 durch die einzelnen Komponenten der 5 Duftmischrollen 51 hindurchfließende Luft nimmt nun mit dem Durchfluß durch jede der Komponenten deren speziellen Duft auf , wodurch sich am Ende der Leitung der dieser Mischung zugeordnete Duft zusammengesetzt hat .

Zur Vermeidung des weiter oben bei Fig . 7 bereits beschriebenen Moments einer möglichen Aromaübertragung werden dabei die schwächsten Duft-Grundkomponenten in der Duftmischrolle 51 angeordnet , in welche die aus dem Tangentialgebläse zugeleitete Luft zuerst hineinfließt , und die stärksten Duftkomponenten in der Duftmischrolle 51, wo die Luft zuletzt , kurz vor dem Austritt aus dem Duftkomposer 50 hindurchfließt .

Um zu vermeiden , daß die jeweils außerhalb der Zuleitung 49 liegenden Geruchskomponenten vorzeitig ihren Duft abgeben , sind diese dabei mit Trennscheiben 47 versehen (in Fig. 15 ist die erste Trennscheibe nicht dargestellt).

Eine andere , nicht dargestellte Möglichkeit, die einzelnen DuftGrundkomponenten ohne relativ aufwendige Ventiltechnik vor einem vorzeitigen Duftverlust zu schützen , wird erreicht , wenn die Duftkomponenten so in den Duftmischrollen 51 gelagert werden , daß sie bei normalen Zimmertemperaturen zwischen 18 und maximal 35 Grad keinen Duft abgeben .

Erst wenn nun die aus dem Tangentialgebläse zugeführte Luft vor dem Eintritt in die Duftmischrollen 51 durch eine kleine Heizspirale geführt und dabei auf eine etwas höhere Temperatur erwärmt wird , z.B. auf 50 Grad , geben die Duftmischrollen 51 durch die erwärmte Luft ihren Duft ab .

Nach dem Austritt aus den Duftmischrollen kann das erwärmte Duftgemisch dann in einer kleinen Kühlschleife wieder auf Zimmertemperatur heruntergekühlt werden oder kühlt auf dem Weg zum Zuschauer je nach Leitungsart und -länge von selbst ab .

Der jeweilige Duft , welcher hierbei hergestellt werden soll , wird nun über einen fünfstelligen Regelimpuls über ein Signalkabel 60 an die Mischrollensteuerung 52 geleitet .

Diese Art der Duftherstellung und Steuerung ermöglicht somit auch eine dezentrale Verwendung des Duftcomposers 50.

Es wäre hierbei z.B. möglich , die Signalleitung zur Mischrollensteuerung 52 an ein Videogerät oder einen Fernseher anzuschließen , wobei der zu bestimmten Szenen zugehörige Regelimpuls dann über ein Funksignal an den Fernseher gesendet wird .

Ein derartiger Duftcomposer wäre somit in der Lage , an sehr vielen verschiedenen Orten zu den verschiedensten Filmen etc . den gleichen , passenden Duft herzustellen , wie das z.B. bei Fernsehsendungen notwendig wäre.

Da der Duftcomposer verschiedene Stärkegrade des jeweiligen Duftes herstellen kann , können z.T. auch graduelle Einleitungen von Duftnoten realisiert werden .
Soweit die Duftmischrollen 51 irgendwann verbraucht sind , können diese dann einzeln oder als kompletter Satz ausgetauscht werden .

Im Verhältnis zu den obigen , in Kinosälen angewendeten , sehr präzisen und außerordentlich variablen Duftverfahren ist der Duftcomposer 50 jedoch in seiner Anwendungsperfektion relativ begrenzt und bleibt auf Anwendungen mit geringen Anforderungen beschränkt .

Ein weiteres und einfacher zu realisierendes Ausführungsbeispiel ergibt sich aus den Fig . 16 und 17 .

Auch dieses Ausführungsbeispiel ist für den dezentralen Gebrauch gedacht . Hier werden die benötigten Duftnoten aber nicht , wie im Duftkomposer 50, gemischt , sondern sind auf einer kleinen Duftdiskette 57 gespeichert .

Die Duftdiskette 57 enthält , ähnlich wie eine miniaturisierte Duftrolle 9 aus dem ersten Ausführungsbeispiel in Fig . 4 , die Duftnoten , die für einen bestimmten Film notwendig sind .

In der Duftdiskette 57 sind die Duftnoten dabei ebenfalls über kleine , nicht dargestellte Duftträger gespeichert . Diese Duftträger können verschiedene , für diesen Zweck geeignete Formen haben .

Das hier nur schematisch dargestellte Duft-Diskettenlaufwerk 55 (Fig . 16) kann nun , im Prinzip ähnlich wie das Diskettenlaufwerk eines herkömmlichen Computers , über den Steuerungsansatz 71 und eine Steuervertiefung 76 der Diskette 57 (Fig . 17) den jeweils geeigneten Duft der Diskette 57 ansteuern . Die Orientierung , wo welcher Duft gespeichert ist , bekommt das Duft-Diskettenlaufwerk 55 in diesem Fall z.B. über die Stellung der Steuervertiefung 76 mitgeteilt .

Im Gehäuse des Duft-Diskettenlaufwerks 55 ist nun, ähnlich wie bei dem Duftcomposer 50 (Fig . 16 , Teil 54), ein niedertourig laufendes und dadurch extrem leises Tangentialgebläse 78 angeordnet (Fig. 17) .

Von diesem Tangentialgebläse 78 wird nun über eine entsprechende Luftleitung 80 ein Luftstrom in die Diskettenduftführung 79 geleitet (ähnlich wie bei dem Duftkomposer in Fig . 15 das Teil 49).
Hierauf werden dann die Duftstoffe aus dem Duftträger , der gerade vor der Duftführung 79 der Film-Duftdiskette 57 plaziert ist, in die Luftleitung 80 geleitet und hiermit schließlich aus dem Gehäuse heraus zum Fernsehzuschauer geführt ( Fig. 16) .

Da auf einer derartigen kleinen Diskette relativ wenig Platz vorhanden ist , werden hier nur die jeweils wichtigsten Duftnoten , die in einem Film auftauchen , gespeichert . Das maximale Fassungsvolumen einer derartigen Duftdiskette könnte bei einer Außenabmessung von z.B. 9 x 9 x 1,5 cm etwa bei 30 Duftnoten liegen .

Das Fassungsvermögen dieser Mini-Duftträger ist dabei sehr begrenzt , so daß eine Duftdiskette 57 , je nach Auslegung,z.B. nur 1-3 mal benutzt werden kann.

Nachdem die Duftdiskette 57 nun in das Duftdisketten-Laufwerk 55 hineingeschoben wurde , steuert das Duftdisketten-Laufwerk 55 zunächst automatisch einen nicht dargestellten sogenannten "Nullpunkt" auf der Duftdiskette 57 an.
Der Nullpunkt befindet sich jeweils vor dem 1. in einem Film eingespielten Duft und ist einfach ein Leerdurchlauf in der Diskettenscheibe , welcher keinen Duft abgibt .
Zu diesem Nullpunkt kehrt die Diskette auch jeweils nach der Beendigung jeder während des Films eingespielten Duftnote zurück , soweit diese im filmischen Geschehen beendet ist .

Auf diese Weise kann das Ende jeder Dufteinspielung ebenfalls vom Diskettenlaufwerk 55 angesteuert werden , ohne daß hierfür zusätzliche Ventile etc. notwendig sind oder das Gebläse 78 jedesmal ein- und ausgeschaltet werden muß .

Das Duftdisketten-Laufwerk 55 ist nun über ein Signalkabel 60 z.B. an einen Videorecorder , einen Fernseher , ein Radio oder ein Diavorführgerät etc. angeschlossen .

Bei der Ausstrahlung z.B. eines Spielfilms oder eines hierfür passenden Musikprogramms , Musicals , einer Theater- oder Ballettaufführung etc . , werden nun die für die Steuerung der Film-Duftdiskette 57 nötigen Steuerbefehle ebenfalls zusammen mit dem gesendeten Fernsehprogramm als nicht hörbare Steuersignale mitgesendet .

Die Signale können nun über ein an das Fernsehgerät anschließbares Zusatzgerät gelesen werden und werden dann von diesem über das kleine Signalkabel 60 dem Duftdisketten-Laufwerk 55 mitgeteilt.

Dieses Zusatzgerät bzw. der Leser für die Duftsignale kann sich dabei auch innerhalb des Duftdisketten-Laufwerks 55 befinden , so daß das Laufwerk 55 über das Signalkabel 60 direkt an einen vorbereiteten Anschluß des Fernsehempfängers angeschlossen werden kann.
Durch die gesendeten Duftsignale werden nun die zu den entsprechenden Szenen des Femseh- oder Videofilms passenden Duftnoten an die Luftleitung 80 abgegeben und z.B. über eine sehr kleine , kurze Leitung zum Zuschauer geführt.
Um die sehr kleinen Duftmengen, die auf einer Duftdiskette 57 gespeichert werden können, vor einem vorzeitigen Verflüchtigen langfristig zu schützen , wird die noch unverkaufte Duftdiskette 57 dabei mit einer Folie eingeschweißt , die erst vor der ersten Benutzung durch den Fernsehzuschauer entfernt wird .

Bei einer entsprechenden Verbreitung des Systems ist es dadurch möglich , vor bestimmten , besonders beliebten Filmen , die über das Fernsehnetz ausgestrahlt werden , die zugehörigen Duftdisketten 57 über den Einzelhandel, wie Zeitschriftenläden etc., zu vertreiben .

Eine weitere Verwendungsmöglichkeit der Film-Duftdiskette 57 besteht darin , daß sie neben der dufttechnischen Ergänzung von Video- und Fernsehsendungen z.B. auch in kleineren öffentlichen Vorführungsälen, z.B. für Musik-, Theater- oder Kinoaufführungen, eingesetzt werden kann , wo es sich nicht rentiert, diese eigens mit einer Duftanlage , wie sie für Kinosäle weiter oben beschrieben wurde, auszustatten.

Auch die Veranstaltungen von Open-Air-Musikkonzerten oder sonstigen Open-Air-Veranstaltungen, die mit Düften begleitet werden sollen ( Freilicht-Kino etc. ), können mit diesem Duftdisketten-System u.U. sinnvoll ausgestattet werden .

In diesem Fall befindet sich auf den dafür vorgesehenen Plätzen jeweils ein kleines Disketten-Laufwerk 55, wo die für Diavortrag, Musikaufführung, Freilichtkino etc. passende Film-Duftdiskette 57 hineingesteckt wird .
Soweit es in diesem Fall bei größeren Veranstaltungen zu aufwendig ist, zu allen Duftdisketten-Laufwerken 55 eigens ein Signalkabel 60 zu legen , können die Diskettenlaufwerke auch z.B. über ein Funksignal angesteuert werden .

## Patentansprüche

1. Verfahren zur Erhöhung der sinnlichen Wahrnehmung von visuellen und/oder akustischen Darbietungen, wobei Zuschauern bzw. Zuhörern synchron zur Darbietung von bestimmten visuellen und/oder akustischen Ereignissen bzw. Szenen dazu Düfte zugeführt werden, dadurch gekennzeichnet, daß die Düfte aus einer mobilen Einheit mit weniger als 1 Liter Luft pro Sekunde zugeführt werden.

2. Verfahren nach Anspruch 1, dadurch gekennzeichnet, daß zwischen 0,3 und 0,005 Liter/Sekunde abgegeben werden.

3. Verfahren nach Anspruch 1 oder 2, dadurch gekennzeichnet, daß die visuellen und/oder akustischen Darbietungen in Kinos, Theatern, Konzert- und Vortragssälen stattfinden sowie bei Diavorträgen, Videos, Fernsehsendungen, Hörspielen und dergleichen.

4. Verfahren nach einem der vorhergehenden Ansprüche, dadurch gekennzeichnet, daß die passenden Düfte aus einem Vorrat von Düften aufgrund wenigstens eines Auswahlsignals individuell zugeführt werden.

5. Verfahren nach einem der vorhergehenden Ansprüche, dadurch gekennzeichnet, daß die Intensität zugeführter Düfte an die individuellen Ereignisse bzw. Darbietungen angepaßt wird.

6. Verfahren nach einem der vorangehenden Ansprüche, dadurch gekennzeichnet, daß der Wechsel von zugeführten Düften mit jeweils zeitlich abnehmender bzw. zunehmender Intensität vorgenommen wird.

7. Verfahren nach einem der vorangehenden Ansprüche, dadurch gekennzeichnet, daß die passenden Düfte jedem Zuschauer bzw. Zuhörer gruppenweise oder separat zu- und/oder abgeführt werden.

8. Verfahren nach einem der vorangehenden Ansprüche, dadurch gekennzeichnet, daß zugeführte Düfte während und/oder nach den zugeordneten Darbietungsereignissen abgeführt werden.

9. Verfahren nach einem der vorhergehenden Ansprüche, dadurch gekennzeichnet, daß die Düfte in Form eines mit Duftstoffen angereicherten Duft-Luft-Gemischstromes zugeführt werden, der mit Bestandteilen sehr niedriger spezifischer Dichte angereichert wird, derart, daß das austretende Duft-Luft-Gemisch leichter als die Umgebungsluft ist.

10. Verfahren nach einem der vorhergehenden Ansprüche, dadurch gekennzeichnet, daß der austretende Duft-Luft-Gemischstrom in eine in Strömungsrichtung fortschreitende rotierende Wirbelbewegung versetzt wird, um so über eine vorgegebene Länge nach dem Austritt einen gerichteten Strömungskanal zu bilden.

11. Verfahren nach Anspruch 1, dadurch gekennzeichnet, daß die Duftstoffe auf festen, stark porösen Materialien gespeichert sind.

12. Verfahren nach Anspruch 1, dadurch gekennzeichnet, daß zur Entfernung auftretender Verunreinigung einer Zuleitung (37) eine Reinigungsflüssigkeit durch die Zuleitung (37) geführt wird.

13. Vorrichtung zur Durchführung des Verfahrens nach Patentanspruch 1, gekennzeichnet durch eine mobile Einheit als Einrichtung zur individuellen Aufnahme und Abgabe verschiedener Düfte und eine zugeordnete Einrichtung zur Ansteuerung bestimmter Duftabgaben und -zuführungen abhängig von bestimmten visuellen und/oder akustischen Ereignissen, wobei weniger als 1 Liter Luft pro Sekunde abgegeben wird.

14. Vorrichtung nach Anspruch 13, dadurch gekennzeichnet, daß zwischen 0,3 und 0,005 Liter/Sekunde abgegeben werden.

15. Vorrichtung nach Anspruch 13 oder 14, dadurch gekennzeichnet, daß die Duftaufnahme- und -abgabeeinrichtung mehrere unterschiedliche Duftvorräte aufweist, die mittels einer Auswahleinrichtung selektiv für die Duftzuführung freigebbar sind.

16. Vorrichtung nach Anspruch 15, dadurch gekennzeichnet, daß die Duftvorräte in freigebbarer fester oder flüssiger Form vorliegen und durch Kontaktierung an einen passierenden Luftstrom abgebbar sind.

17. Vorrichtung nach Anspruch 15, dadurch gekennzeichnet, daß die Duftvorräte gasförmig oder in Fluid- bzw. Aerosolform vorliegen und unter Druck freigebbar sind.

18. Vorrichtung nach einem der Ansprüche 15 bis 17, dadurch gekennzeichnet, daß die Duftvorräte in wenigstens einer Duftschaltbox, einer Duftdiskette oder einer Duftrollenanordnung enthalten sind.

19. Vorrichtung nach einem der Ansprüche 13 bis 18, dadurch gekennzeichnet, daß die Duftaufnahme- und -abgabeeinrichtung eine Duftintensitätsregelung aufweist.

20. Vorrichtung nach einem der Ansprüche 13 bis 19, dadurch gekennzeichnet, daß die Duftaufnahme- und -abgabeeinrichtung für das Einbringen des jeweiligen Duftes in einen Luftstrom ein spiralförmiges Leitungssystem mit Ein- und Auslaßsteuerelementen mit zugeordneter Bypassleitung aufweist.

21. Vorrichtung nach einem der Ansprüche 13 bis 19, dadurch gekennzeichnet, daß die Duftaufnahme- und -abgabeeinrichtung für das Einbringen des jeweiligen Duftes in einen Luftstrom wenigstens zwei Kontaktierflächen für Lüftstromteile aufweist.

22. Vorrichtung nach einem der Ansprüche 13 bis 19, dadurch gekennzeichnet, daß die Duftaufnahme- und -abgabeeinrichtung als Duftmischer für mehrere Duftgrundkomponenten ausgebildet ist, die in durch Einzelluftströme ansteuerbaren Duftmischrollen vorgesehen sind.

23. Vorrichtung nach einem der Ansprüche 13 bis 22, dadurch gekennzeichnet, daß die mobile Einheit eine Duftdiskette oder ein Duftmischer ist.

24. Vorrichtung nach Anspruch 23, dadurch gekennzeichnet, daß die Reihenfolge der Kontaktierung der Duftmischrollen abhängig von deren Duftstärke derart festgelegt ist, daß die stärkste Duftkomponente zuletzt kontaktiert wird, und daß der Duftmischer ein Gebläse zur Luftstromerzeugung aufweist.

25. Vorrichtung nach einem der Ansprüche 13 bis 24, dadurch gekennzeichnet, daß die Duftvorräte gegen Duftverlust durch zu öffnende Abschlußeinrichtungen und/oder temperaturabhängige Duftfreisetzung geschützt sind.

26. Vorrichtung nach einem der Ansprüche 13 bis 25, dadurch gekennzeichnet, daß die Einrichtung zur Ansteuerung individueller Duftabgaben und -zuführungen eine Erfassungseinrichtung für individuelle ereignissynchrone Duftsteuersignale aufweist.

27. Vorrichtung nach Anspruch 26, dadurch gekennzeichnet, daß die Erfassungseinrichtung zur Aufnahme von optischen und/oder elektrischen Duftsteuersignalen ausgebildet ist, welche kodierte Information über Duftart und/oder Duftzusammensetzungen und/oder Duftvorratsalter und/oder Häufigkeit der Verwendung des Duftvorrates sowie Duftzuführdauer enthalten.

28. Vorrichtung nach Anspruch 27, dadurch gekennzeichnet, daß die Duftsteuersignale weiterhin Informationen zur Duftintensität, zum Duftzuführbeginn und -zuführende sowie zu einem Duftwechsel enthalten.

29. Vorrichtung nach einem der Ansprüche 26 bis 28, dadurch gekennzeichnet, daß als Duftsteuersignale kodierte Filmspuren oder ereignisgekoppelte Signalgeber vorgesehen sind.

30. Vorrichtung nach einem der Ansprüche 13 bis 29, dadurch gekennzeichnet, daß zur Erzielung eines gerichteten rotierenden Wirbelstromes des austretenden Duft-Luft-Gemisches im Bereich der Austrittsöffnungen der Austrittsvorrichtung wendelartige Elemente, insbesondere wendelförmig angeordnete Röhrchen vorgesehen sind.

31. Vorrichtung nach Anspruch 30, dadurch gekennzeichnet, daß jeweils zwei Austrittsöffnungen in der Austrittsvorrichtung so angeordnet sind, daß die beiden rotierenden Duft-Luft-Gemischströme eine gegenläufige Drehbewegung ausführen und sich in ihrer Drehbewegung unterstützen, um so zusätzlich einen resultierenden tangentialen, gerichteten Duft-Luft-Gemischstrom zu erzeugen.

32. Vorrichtung nach Anspruch 13, dadurch gekennzeichnet, daß Duftstoffe auf festen, stark porösen Materialien gespeichert sind.

33. Vorrichtung nach Anspruch 13, dadurch gekennzeichnet, daß sie eine Pumpeinrichtung zur Spülung einer Zuleitung (37) mit einer Reinigungsflüssigkeit aufweist.

## Claims

1. Process for intensifying the sensorial perception of visual and/or acoustic presentations, wherein an audience is supplied in synchronism with the presentation of specific visual and/or acoustic events or scenes with scents, characterized in that the scents are supplied from a mobile unit with less than 1 liter of air per second.

2. Process according to claim 1, characterized in that between 0.3 and 0.005 liter/second is emitted.

3. Process according to claim 1 or 2, characterized in that the visual and/or acoustic presentations take place in cinemas, theaters, concert and lecture halls as well as in slide lectures, videos, television presentations, radio plays and the like.

4. Process according to one of the preceding claims, characterized in that the respective scents are individually supplied from a scent reservoir in response to at least one selection signal.

5. Process according to one of the preceding claims, characterized in that the intensity of supplied scents is adjusted to the individual events or presentations.

6. Process according to one of the preceding claims, characterized in that the change of supplied scents is effected with the intensity thereof decreasing or increasing with time.

7. Process according to one of the preceding claims, characterized in that the respective scents are supplied to and/or withdrawn from the audience separately or in groups.

8. Process according to one of the preceding claims, characterized in that supplied scents are withdrawn during and/or after the associated events of the presentation.

9. Process according to one of the preceding claims, characterized in that the scents are supplied in the form of a scent-enriched scent/air mixture stream enriched with ingredients of very low specific gravity so that the emerging scent/air mixture is lighter than the ambient air.

10. Process according to one of the preceding claims, characterized in that a rotary whirling motion progressing in the direction of flow is imparted on the emerging scent/air mixture flow to thus form a directional flow channel over a predetermined length after discharge.

11. Process according to claim 1, characterized in that the scents are stored on solid, highly porous materials.

12. Process according to claim 1, characterized in that, in order to remove dirt accumulating in a conduit (37), a cleaning fluid is supplied through said conduit (37).

13. Device for carrying out the process according to claim 1, characterized by a mobile unit as arrangement for individually storing and releasing different scents and an associated arrangement for activating the release and supply of specific scents in response to specific visual and/or acoustic events, with less than 1 liter of pair per second being emitted.

14. Device according to claim 13, characterized in that between 0.3 and 0.005 liter/second is emitted.

15. Device according to claim 13 or 14, characterized in that the scent storing and releasing arrangement comprises several different scent reservoirs which are selectively releasable for scent supply by means of a selection means.

16. Device according to claim 15, characterized in that the scents are stored in releasable solid or liquid form and can be released into a passing air flow by way of contact.

17. Device according to claim 15, characterized in that the scents are stored in the form of gas or in the form of a fluid or aerosol and are releasable under pressure.

18. Device according to one of claims 15 to 17, characterized in that the scents are stored in at least one scent switch box, a scent disc or a scent roll arrangement.

19. Device according to one of claims 13 to 18, characterized in that the scent storing and releasing arrangement comprises a scent intensity controlling means.

20. Device according to one of claims 13 to 19, characterized in that the scent storing and releasing arrangement comprises a spiral-shaped conduit system including inlet and outlet control elements and an associated by-pass conduit for introducing the respective scent into an air flow.

21. Device according to one of claims 13 to 19, characterized in that the scent storing and releasing arrangement comprises at least two contact surfaces for air flow portions for introducing the respective scent into an air flow.

22. Device according to one of claims 13 to 19, characterized in that the scent storing and releasing arrangement is designed as scent mixer for several basic scent components provided in scent mixing rolls activatable by single air flows.

23. Device according to one of claims 13 to 22, characterized in that the mobile unit is a scent disc or a scent mixer.

24. Device according to claim 23, characterized in that the sequence of contacts with the scent mixing rolls is determined as a function of the scent intensity thereof so that the strongest scent component is contacted last, and the scent mixer comprises a blower to generate the air flow.

25. Device according to one of claims 13 to 24, characterized in that the scent reservoirs are protected against scent loss by openable sealing means and/or by temperature-dependent scent release.

26. Device according to one of claims 13 to 25, characterized in that the arrangement for activating the release and supply of individual scents comprises a detecting means for individual scent control signals in synchronism with respective events.

27. Device according to claim 26, characterized in that the detecting means is designed for receiving optical and/or electric scent control signals containing encoded information on the type of scent and/or scent compositions and/or age of scent reservoirs and/or frequency of use of the scent reservoir and on the duration of scent supply.

28. Device according to claim 27, characterized in that the scent control signals further contain information on the scent intensity, the beginning and end of scent supply, and the change of scents.

29. Device according to one of claims 26 to 28, characterized in that encoded film tracks or event-coupled signal transmitters are provided as scent control signals.

30. Device according to one of claims 13 to 29, characterized in that helical elements, in particular small helically arranged ducts, are provided to generate a directional rotating whirling stream of emerging scent/air mixture in the area of the outlet openings of an outlet means.

31. Device according to claim 30, characterized in that two outlet openings are arranged in each outlet means such that the two rotating scent/air mixture streams perform rotary motions in opposite directions and sustain each other in said rotary motions in order to additionally generate a tangential directional scent/air mixture stream.

32. Device according to claim 13, characterized in that scents are stored on solid, highly porous materials.

33. Device according to claim 13, characterized in that it comprises a pump means for flushing a conduit (37) with a cleaning fluid.

## Revendications

1. Procédé destiné à accroître la perception sensorielle de représentations visuelles et/ou acoustiques, des parfums adaptés étant envoyés aux spectateurs ou aux auditeurs, de manière synchrone avec la représentation d'évènements ou de scènes visuelles et/ou acoustiques déterminés, caractérisé en ce que les parfums sont envoyés à partir d'une unité mobile avec moins de 1 litre/seconde d'air.

2. Procédé selon la revendication 1, caractérisé en ce que la quantité d'air délivrée est comprise entre 0,3 et 0,005 litre/seconde.

3. Procédé selon la revendication 1 ou 2, caractérisé en ce que les représentations visuelles et/ou acoustiques ont lieu dans des cinémas, des théâtres, des salles de concert et de représentation et, au cours de présentations de diapositives, de vidéos, d'émissions de télévision, de concerts et analogues.

4. Procédé selon l'une des revendications précédentes, caractérisé en ce que les parfums adaptés sont envoyés individuellement à partir d'une réserve de parfums, sur la base d'au moins un signal de sélection.

5. Procédé selon l'une des revendications précédentes, caractérisé en ce que l'intensité des parfums envoyés est adaptée aux évènements ou représentations individuelles.

6. Procédé selon l'une des revendications précédentes, caractérisé en ce que le changement de parfum envoyé est effectué avec, dans chaque cas, une intensité décroissante ou croissante dans le temps.

7. Procédé selon l'une des revendications précédentes, caractérisé en ce que les parfums adaptés sont envoyés à chaque spectateur ou auditeur, et/ou éloignés de celui-ci, par groupe ou séparément.

8. Procédé selon l'une des revendications précédentes, caractérisé en ce que les parfums envoyés sont éloignés pendant et/ou après les évènements correspondants de la représentation.

9. Procédé selon l'une des revendications précédentes, caractérisé en ce que les parfums sont envoyés sous la forme d'un courant mélangé de parfums et d'air, enrichi de substances parfumées, qui est enrichi de constituants de très faible masse spécifique, de manière que le mélange d'air et de parfums sortant soit plus léger que l'air ambiant.

10. Procédé selon l'une des revendications précédentes, caractérisé en ce que le courant mélangé d'air et de parfum sortant est animé d'un mouvement tourbillonnaire, tournant en progressant dans le sens de l'écoulement, afin de former ainsi un canal d'écoulement dirigé, sur une longueur donnée, après la sortie.

11. Procédé selon la revendication 1, caractérisé en ce que les substances parfumées sont stockées sur des matériaux solides fortement poreux.

12. Procédé selon la revendication 1, caractérisé en ce que, pour éliminer une impureté entrant dans une conduite (37), on fait passer un liquide de nettoyage à travers la conduite (37).

13. Dispositif pour la mise en oeuvre du procédé selon la revendication 1, caractérisé par une unité mobile comme dispositif pour la réception et la distribution individuelle de différents parfums et un dispositif associé pour la commande de dégagement et d'envois de parfums déterminés, en fonction d'évènements visuels et/ou acoustiques déterminés, dans lequel la quantité d'air délivrée est inférieure à 1 litre/seconde.

14. Dispositif selon la revendication 13, caractérisé en ce que la quantité d'air délivrée est comprise entre 0,3 et 0,005 litre/seconde.

15. Dispositif selon la revendication 13 ou 14, caractérisé en ce que le dispositif de réception et de distribution de parfums présente plusieurs réserves de parfum différentes, qui peuvent être libérées de manière sélective pour l'envoi de parfum, au moyen d'un dispositif de sélection.

16. Dispositif selon la revendication 15, caractérisé en ce que les réserves de parfum se trouvent sous une forme solide ou liquide libérable et peuvent être délivrées par contact avec un courant d'air passant.

17. Dispositif selon la revendication 15, caractérisé en ce que les réserves de parfum se trouvent sous forme de gaz ou sous forme fluide ou d'aérosol et peuvent être libérées sous pression.

18. Dispositif selon l'une des revendications 15 à 17, caractérisé en ce que les réserves de parfum sont contenues dans au moins une boîte de commande de parfums, une disquette à parfums ou un dispositif à rouleaux de parfums.

19. Dispositif selon l'une des revendications 13 à 18, caractérisé en ce que le dispositif de réception et de distribution de parfums présente un réglage d'intensité de parfum.

20. Dispositif selon l'une des revendications 13 à 19, caractérisé en ce que le dispositif de réception et de distribution de parfums comporte, pour l'introduction du parfum respectif dans un courant d'air, un système de conduite en spirale pourvu d'éléments de commande d'entrée et de sortie et d'une conduite de dérivation correspondante.

21. Dispositif selon l'une des revendications 13 à 19, caractérisé en ce que le dispositif de réception et de distribution de parfums comporte, pour l'introduction du parfum respectif dans un courant d'air, au moins deux surfaces de contact avec des parties de courant d'air.

22. Dispositif selon l'une des revendications 13 à 19, caractérisé en ce que le dispositif de réception et de distribution de parfums est conformé en mélangeur de parfums à partir de plusieurs composants de base, qui sont prévus dans des rouleaux de mélange de parfums pouvant être commandés par des courants d'airs individuels.

23. Dispositif selon l'une des revendications 13 à 22, caractérisé en ce que l'unité mobile est une disquette à parfums ou un mélangeur de parfums.

24. Dispositif selon la revendication 23, caractérisé en ce que la mise en contact successive des rouleaux de mélange de parfums, en fonction de leur intensité, est fixée de manière que le composant de parfum le plus intense soit mis en contact en dernier, et en ce que le mélangeur de parfums comporte un ventilateur pour la production d'un courant d'air.

25. Dispositif selon l'une des revendications 13 à 24, caractérisé en ce que les réserves de parfum sont protégées contre une perte de parfum par des dispositifs de fermeture s'ouvrant et/ou par un dégagement de parfum en fonction de la température.

26. Dispositif selon l'une des revendications 13 à 25, caractérisé en ce que le dispositif de commande de dégagement et d'envois individuels de parfums comporte un dispositif de détection de signaux de commande de parfums individuels, synchrone aux évènements.

27. Dispositif selon la revendication 26, caractérisé en ce que le dispositif de détection est conformé pour la réception de signaux optiques et/ou électriques de commande de parfums, qui contiennent une information codée relative au type de parfum et/ou aux compositions de parfums et/ou à l'âge de la réserve de parfum et/ou à la fréquence de l'utilisation de la réserve de parfum, ainsi qu'à la durée d'envoi du parfum.

28. Dispositif selon la revendication 27, caractérisé en ce que les signaux de commande de parfums contiennent en outre des informations relatives à l'intensité du parfum, au début et à la fin de l'envoi du parfum ainsi qu'à un changement de parfum.

29. Dispositif selon l'une des revendications 26 à 28, caractérisé en ce que sont prévus, comme signaux de commande de parfum, des pistes de film ou des capteurs de signaux couplés à un événement.

30. Dispositif selon l'une des revendications 13 à 29, caractérisé en ce que, pour obtenir un courant tourbillonnaire tournant dirigé du mélange d'air et de parfums sortant, des éléments en hélice, en particulier de petits tubes disposés en forme d'hélice, sont prévus dans la région des orifices de sortie du dispositif de sortie.

31. Dispositif selon la revendication 30, caractérisé en ce que deux orifices de sortie sont à chaque fois disposés dans le dispositif de sortie, de manière que les deux courants mélangés d'air et de parfum tournant exécutent un mouvement de rotation en sens contraires et se soutiennent dans leur mouvement de rotation, afin de produire ainsi, de plus, un courant mélangé d'air et de parfums résultant, dirigé tangentiellement.

32. Dispositif selon la revendication 13, caractérisé en ce que les substances parfumées sont stockées sur des matériaux solides fortement poreux.

33. Dispositif selon la revendication 13, caractérisé en ce qu'il est muni d'une installation de pompage pour le rinçage d'une conduite (37) avec un liquide de nettoyage.
